# EUROPEAN PATENT SPECIFICATION

(11) **EP 1 718 745 B1**
(45) Date of publication and mention of the grant of the patent: **19.11.2008**
(21) Application number: 05715582.2
(22) Date of filing: 26.02.2005
(51) Int. Cl.: C12N 15/10, C12N 1/20, C12N 15/65

(54) **GENERATION OF RECOMBINANT GENES IN PROKARYOTIC CELLS BY USING TWO EXTRACHROMOSOMAL ELEMENTS**
ERZEUGUNG REKOMBINANTER GENE IN PROKARYONTISCHEN ZELLEN UNTER VERWENDUNG VON ZWEI EXTRACHROMOSOMALEN ELEMENTEN
PRODUCTION DE GENES DE RECOMBINAISON DANS DES CELLULES PROCARYOTES PAR LE BIAIS DE DEUX ELEMENTS EXTRACHROMOSOMIQUES

(30) Priority: 26.02.2004 EP 04360018
(43) Date of publication of application: 08.11.2006
(73) Proprietor: Mixis France S.A., 75014 Paris (FR)
(72) Inventor: RODRIGUEZ-GOMEZ, Ana, E-39570 Potes (ES); GALIC, Tatjana, 10000 Zagreb (HR); PETIT, Marie-Agnès, F-75010 Paris (FR); MATIC, Ivan, F-92100 Boulogne-Billancourt (FR); RADMAN, Miroslav, F-94250 Gentilly (FR)
(74) Representative: Crawley, Karen Anne
(86) International application number: PCT/EP2005/002066
(87) International publication number: WO 2005/083079

(56) References cited:
- TAYLOR R K ET AL: "BROAD-HOST-RANGE VECTORS FOR DELIVERY OF TNPHOA: USE IN GENETIC ANALYSIS OF SECRETED VIRULENCE DETERMINANTS OF VIBRO CHOLERAE" 1 April 1989 (1989-04-01), JOURNAL OF BACTERIOLOGY, WASHINGTON, DC, US, PAGE(S) 1870-1878 , XP000654720 ISSN: 0021-9193 figure 2
- GINNS C A ET AL: "Colonization of the respiratory tract by a virulent strain of avian Escherichia coli requires carriage of a conjugative plasmid" INFECTION AND IMMUNITY, vol. 68, no. 3, March 2000 (2000-03), pages 1535-1541, XP002332527 ISSN: 0019-9567
- VAGNER V ET AL: "A VECTOR FOR SYSTEMATIC GENE INACTIVATION IN BACILLUS SUBTILIS" MICROBIOLOGY, SOCIETY FOR GENERAL MICROBIOLOGY, READING, GB, vol. 144, no. 11, November 1998 (1998-11), pages 3097-3104, XP008026300 ISSN: 1350-0872
- ANONYMOUS: "Transposon-based DNA Sequencing, Mutagenesis and Linker Scanning Systems" 2002, NEW ENGLAND BIOLABS CATALOG 2002-2003 , XP002332528 page 154 - page 155
- DATSENKO KIRILL A ET AL: "One-step inactivation of chromosomal genes in Escherichia coli K-12 using PCR products" PROCEEDINGS OF THE NATIONAL ACADEMY OF SCIENCES OF USA, NATIONAL ACADEMY OF SCIENCE. WASHINGTON, US, vol. 97, no. 12, 6 June 2000 (2000-06-06), pages 6640-6645, XP002210218 ISSN: 0027-8424
- SCHNEIDER W P ET AL: "PROCEDURE FOR PRODUCTION OF HYBRID GENES AND PROTEINS AND ITS USE IN ASSESSING SIGNIFICANCE OF AMINO-ACID DIFFERENCES IN HOMOLOGOUS TRYPTOPHAN SYNTHETASE ALPHA POLY PEPTIDES" PROCEEDINGS OF THE NATIONAL ACADEMY OF SCIENCES OF THE UNITED STATES OF AMERICA, vol. 78, no. 4, 1981, pages 2169-2173, XP002332529 ISSN: 0027-8424
- DEGRYSE E: "In vivo intermolecular recombination in Escherichia coli: application to plasmid constructions" GENE, ELSEVIER BIOMEDICAL PRESS. AMSTERDAM, NL, vol. 170, no. 1, 17 April 1996 (1996-04-17), pages 45-50, XP004042868 ISSN: 0378-1119
- WANG P L: "CREATING HYBRID GENES BY HOMOLOGOUS RECOMBINATION" DISEASE MARKERS, WILEY, CHICHESTER, GB, vol. 16, no. 1/2, April 2000 (2000-04), pages 3-13, XP001064283 ISSN: 0278-0240

### SEQUENCE LISTING

<110> Mixis France S.A.
<120> Generation of recombinant genes in prokaryotic cells by using two extrachromosomal elements
<130> 18249
<140>
   <141>
<160> 17
<170> PatentIn Ver. 2.1
<210> 1
   <211> 31
   <212> DNA
   <213> Artificial Sequence
<220>
   <223> Description of Artificial Sequence: Primer
<400> 1
   gcgcagtact cctcactcgg ggcggaaaag g 31
<210> 2
   <211> 29
   <212> DNA
   <213> Artificial Sequence
<220>
   <223> Description of Artificial Sequence: Primer
<400> 2
   gcgcaggtcc cgtttgagct caggccgcg 29
<210> 3
   <211> 29
   <212> DNA
   <213> Artificial Sequence
<220>
   <223> Description of Artificial Sequence: Primer
<400> 3
   gcggatccat gcctgcaggg acgcctttg 29
<210> 4
   <211> 33
   <212> DNA
   <213> Artificial Sequence
<220>
   <223> Description of Artificial Sequence: Primer
<400> 4
   gcgcagggcc tggtcacgat gctgtacttt gtg 33
<210> 5
   <211> 28
   <212> DNA
   <213> Artificial Sequence
<220>
   <223> Description of Artificial Sequence: Primer
<400> 5
   gcggatcctg ttagccacca aggtacca 28
<210> 6
   <211> 31
   <212> DNA
   <213> Artificial Sequence
<220>
   <223> Description of Artificial Sequence: Primer
<400> 6
   gcgcagggcc tttagccacc aatgatgatg c 31
<210> 7
   <211> 27
   <212> DNA
   <213> Artificial Sequence
<220>
   <223> Description of Artificial Sequence: Primer
<400> 7
   gcggatcccc ctttaccaaa ccaatac 27
<210> 8
   <211> 29
   <212> DNA
   <213> Artificial Sequence
<220>
   <223> Description of Artificial Sequence: Primer
<400> 8
   gcgcagggcc taagggttgg gcgattttg 29
<210> 9
   <211> 31
   <212> DNA
   <213> Artificial Sequence
<220>
   <223> Description of Artificial Sequence: Primer
<400> 9
   gcgcagtact atgcctgcag ggacgccttt g 31
<210> 10
   <211> 33
   <212> DNA
   <213> Artificial Sequence
<220>
   <223> Description of Artificial Sequence: Primer
<400> 10
   tcgcgggacc tggtcacgat gctgtacttt gtg 33
<210> 11
   <211> 30
   <212> DNA
   <213> Artificial Sequence
<220>
   <223> Description of Artificial Sequence: Primer
<400> 11
   gcgcagtact gggcgaaccc ggagcctcat 30
<210> 12
   <211> 22
   <212> DNA
   <213> Artificial Sequence
<220>
   <223> Description of Artificial Sequence: Primer
<400> 12
   aagaaggagt gattacatga ac 22
<210> 13
   <211> 19
   <212> DNA
   <213> Artificial Sequence
<220>
   <223> Description of Artificial Sequence: Primer
<400> 13
   cgcatctcgg gcagcgttg 19
<210> 14
   <211> 20
   <212> DNA
   <213> Artificial Sequence
<220>
   <223> Description of Artificial Sequence: Primer
<400> 14
   gcagatccgg aacataatgg 20
<210> 15
   <211> 20
   <212> DNA
   <213> Artificial Sequence
<220>
   <223> Description of Artificial Sequence: Primer
<400> 15
   tgtcggcaga atgcttaatg 20
<210> 16
   <211> 20
   <212> DNA
   <213> Artificial Sequence
<220>
   <223> Description of Artificial Sequence: Primer
<400> 16
   cactatcgac tacgcgatca 20
<210> 17
   <211> 19
   <212> DNA
   <213> Artificial Sequence
<220>
   <223> Description of Artificial Sequence: Primer
<400> 17
   gcttccccat gataagagc 19

## Description

The present invention relates in general to in vivo methods for generating and detecting recombinant DNA sequences in prokaryotic cells, in particular bacteria, by using two different extrachromosomal elements and extrachromosomal elements, in particular plasmids that can be used for conducting the inventive methods. DNA sequences for which these methods are relevant include protein-encoding and non-coding sequences.

Evolution is a continuous process of genetic variation and phenotypic selection. The genetic diversity of a population can be amplified by creating new mutant combinations improving the performance of individuals within the population. The directed evolution of microorganisms has been accomplished traditionally through the process of classical strain improvement, by sequential random mutagenesis and screening.

Directed evolution has so far been used almost exclusively as a tool for engineering proteins. By mutation techniques such as site-directed mutagenesis, cassette mutagenesis, random mutagenesis, and error prone PCR variants of protein functions have been generated and the libraries thus produced have been screened for their ability to perform a specific function. Recursive application of this procedure has been used successfully for the modification of physical and catalytic properties of enzymes such as pH optima, thermotolerance, solvent stability, stereoselectivity, catalytic activity and substrate specificity as well as toxicity resistance mechanisms in bacteria and the host range and stability of viruses.

Traditional mutagenesis approaches for evolving new properties in enzymes have a number of limitations. These approaches are only applicable to genes or sequences that have been cloned and functionally characterized and that have a discrete function. Also, the traditional mutagenesis approaches can only explore a very limited number of the total number of permutations, even for a single gene. However, under certain circumstances it might be necessary to modify not only one gene, but additional genes, in order to express a protein with new properties. Such additional genes can be for example genes that cooperatively confer a single phenotype or genes that have a role in one or more cellular mechanisms such as transcription, translation, post-translational modifications, secretion or proteolytic degradation of a gene product. Attempting to individually optimize all of the genes having such function by traditional mutagenesis approaches would be a virtually impossible task.

Most of the problems associated with conventional mutagenesis approaches can be overcome by recombination approaches which entail randomly recombining different sequences of functional genes, enabling the molecular mixing of naturally similar or randomly mutated genes. In comparison to conventional mutagenesis with recombination, the probability of obtaining mutants with improved phenotype is significantly higher. The main advantages of recombination approaches over conventional DNA manipulation technologies are in particular the experimental simplicity and the freedom from DNA sequence-imposed limitations.

Much of that what is known about recombination processes has come from studies of simple, unicellular organisms such as bacteria. The study of recombination in such organisms has the advantage of the ease of manipulation of DNA sequences and the possibility of studying specific recombination events induced synchronously in a large proportion of cells. Equally important is the growing conviction that the processes studied in microorganisms are identical or similar in most respects to the ways in which mammalian cells, e.g. human cells, generate genetic diversity. Moreover, defining these mechanisms has taken on added importance in the quest to develop more efficient mechanisms of gene targeting and gene replacement in mammalian cells.

Datsenko et al, Proceedings of the National Academy of Sciences of USA, National Academy of Science. Washington, US, Vol. 97, No. 12, 6 June 2000, 6640-6645 discloses a method to disrupt chromosomal genes in *Escherichia coli* in which PCR primers provide the homology to the targeted genes. The recombination procedure requires the phage λ Red recombinase.

Zhang et al (1998) Nature Genetics Vol 20, 123-128 discloses homologous recombination using RecE and RecT as a method of engineering DNA unlimited by the disposition of restriction endonuclease cleavage sites or the size of the target DNA.

EP0687730 discloses a method of transforming plants whereby a regenerated transgenic plant containing desired genes introduced thereinto is prepared at a high efficiency and which permits the preparation in the subsequent generation of a transgenic plant containing the desired genes but not the drug-resistant genes used as the selection marker. The method involves the formation of a cointegrant plasmid in Agrobacterium tumerofaciens.

Although numerous different systems for effecting recombination in prokaryotic cells exists most of these do not allow an easy and reliable detection of newly recombined DNA sequences. Therefore, there is still in the art a demand for efficient prokaryotic test systems, which in particular allow a rapid and simple detection of recombinants and/or a selection of recombinants under selective pressure.

Therefore, the technical problem underlying the present invention is to provide improved methods and means for a simple and efficient generation of recombinant mosaic genes in prokaryotic cells, in particular for screening and detecting such recombinant sequences.

The present invention solves this underlying technical problem by providing a process for generating and detecting recombinant DNA sequences in procaryotes comprising the steps of:
a) generating a first prokaryotic cell containing a recipient DNA molecule, which comprises a first DNA sequence to be recombined and which can autonomously replicate in the prokaryotic cell, and a donor DNA molecule, which comprises a second DNA sequence to be recombined and at least a first marker sequence encoding a gene product and which cannot autonomously replicate in the prokaryotic cell,
b) cultivating the first prokaryotic cell under selective conditions which only allow the growth and/or propagation of the cell if the gene product of the first marker sequence is expressed, and
c) isolating a second prokaryotic cell grown and/or propagated under selective conditions and containing a hybrid DNA molecule with the at least first marker sequence and a first and a second recombined DNA sequences due to recombination between the first and the second DNA sequences,
wherein the prokaryotic cell is transiently or permanently deficient in the mismatch repair system.

The present invention provides a prokaryotic system to screen for recombination events between at least two diverging, or heterologous, DNA sequences or recombination substrates in vivo. The inventive system allows the generation of new advantageous DNA sequences with improved properties in a fast and efficient way from at least two diverging DNA sequences by a process involving an in vivo exchange of DNA from two extrachromosomal elements containing the two DNA sequences to be recombined. The two extrachromosomal elements showing no homology in their nucleotide sequences are introduced into a prokaryotic host cell in the form of a recipient DNA molecule and a donor DNA molecule. The recipient molecule can autonomously replicate in the host, whereas the donor molecule does not have the ability to replicate. However, the donor molecule contains at least one unique protein-encoding marker sequence such as an antibiotic resistance marker or nutritional marker which is neither present in the genome of the host cell nor in the recipient molecule. After the introduction of both extrachromosomal elements into the prokaryotic host cell the cell is cultivated under conditions which force the recombination between the two heterologous genes. These conditions can include for example a cultivation of the cell in the presence of an antibiotic to which the cell is normally sensitive or a cultivation of the cell in a medium lacking an essential nutrient, which the cell cannot synthesize itself and which therefore has to be externally supplied. Under the cultivation conditions applied the cell can only grow and propagate, i.e. divide, if the gene product of the respective marker sequence is expressed. A prerequisite for the expression of the marker sequence is, however, that the non-replicative donor molecule and the replicative recipient molecule form a co-integrate which can replicate from the origin of the recipient molecule and thus ensures the maintenance of the marker sequence. The formation of this co-integrate results from recombination between the two recombination substrates leading to the generation of new DNA molecules whose sequences differ from that of the parental DNA sequences. Host cells grown and propagated under the selective conditions applied therefore contain new recombined DNA sequences. The inventive process therefore provides an easy and quick selection system to identify recombinant DNA sequences. With the inventive process a large library of recombined, mutated DNA sequences can be easily generated, and variants that have acquired a desired function can then be identified by using an appropriate selection or screening system.

The study of recombination processes in a simple, unicellular organism such as a prokaryote has the obvious advantage of the ease of manipulation of DNA sequences and the possibility of studying specific recombination events induced synchronously in a large proportion of cells. Furthermore, over the last few decades a wealth of expertise has been accumulated both in the fermentation technology and the basic genetics of prokaryotic organisms. Another major advantage of prokaryotic host cells relates to their very short doubling times. Thus, by the use of prokaryotic host cells it is possible to conduct many rounds of cell division and thus many rounds of recombination and to create a plurality of new recombinant DNA sequences within a short time.

The inventive process can be conducted either in wild-type or mismatch repair defective prokaryotic cells. The processes by which damaged DNA is repaired and the mechanisms of genetic recombination are intimately related; and it is known that the mismatch repair machinery has inhibitory effects on the recombination frequency between divergent sequences, i.e. homeologous recombination. Mutations of the mismatch repair system therefore greatly enhance the overall frequency of recombination events in prokaryotic cells. On the other hand, it is known that prokaryotic wild-type cells have a mismatch repair-dependent recombination mechanism, which is based on distantly spaced mismatches in two recombination substrates. Depending on the DNA sequences to be recombined, either wild-type or mismatch repair-defective prokaryotic cells can be used to obtain recombined sequences.

The inventive process for generating and detecting recombined DNA sequences has the advantage that greatly diverging DNA sequences can be recombined. It was unexpectedly found by the inventors that sequences with a high degree of overall divergence and which only share very short stretches of homology or identity can be recombined. An analysis of recombined sequences revealed that the longest stretches of identity in which recombination occurred comprise only 18-22 nucleotides. Most of the recombination events occurred in homologous stretches with a length of 10-15 nucleotides. In some cases recombination occurred in stretches with a length of only 4-5 nucleotides.

Therefore, the inventive process allows the recombination of DNA sequences derived from different prokaryotic species or different prokaryotic genera in order to create recombinant DNA sequences with advantageous features.

The inventive process for generating and detecting recombined DNA sequences has the advantage that more than two diverging sequences can be recombined, whereby the two diverging sequences to be recombined can be either pre-selected or non-selected sequences.

For example a variety of diverged DNA sequences up to a whole gene library can be inserted into the recipient as well as the donor DNA molecules. Afterwards the individual diverged DNA sequences can be recombined by chance with themselves. It is also possible to insert a first set of diverged DNA sequences up to a whole gene library only into the recipient DNA molecules and to insert a second set of diverged DNA sequences up to a whole gene library into the donor DNA molecules or vice versa and then to recombine the two sets by chance with each other. In both cases there is no selection as to which pair of diverged DNA sequences will be recombined.

However, it is also possible to recombine several, preferably pre-selected, diverging sequences in a stepwise manner, whereby in each step a selection is done as to which pair of diverged DNA sequences shall be recombined. If, for example, three diverging DNA sequences shall be recombined, than in the first step first prokaryotic cells are generated, whereby for example, a recipient DNA molecule with a first DNA sequence to be recombined and a donor DNA molecule with a second DNA sequence to be recombined are introduced into bacterial cells of a given species. These prokaryotic cells are cultivated under selective conditions such, that only cells are grown and propagated, in which the respective recipient and donor DNA molecules have formed a hybrid molecule enabling the expression of the marker sequence of the donor molecule and recombination between the two DNA sequences to be recombined. Thus second prokaryotic cells are obtained containing a hybrid molecule with a first and a second recombined DNA sequences due to recombination. The first and second recombined sequences are isolated and one of them is inserted for example into a recipient molecule, whereas the third DNA sequence to be recombined is inserted into the donor molecule. Then, in the next step, the recipient molecule comprising one of the recombined sequences and the donor molecule comprising the third DNA sequence to be recombined are introduced again in a prokaryotic host cell and subjected another round of recombination.

If, for example, four diverging DNA sequences shall be recombined, then in the first step two different sets of first prokaryotic cells are generated. For example, a first set of first prokaryotic cells can be generated by introducing a recipient DNA molecule with a first DNA sequence to be recombined and a donor DNA molecule with a second DNA sequence to be recombined into prokaryotic cells. Likewise a second set of first prokaryotic cells can be generated by introducing a recipient DNA molecule with a third DNA sequence to be recombined and a donor DNA molecule with a fourth DNA sequence to be recombined into cells of the same species. Each set of prokaryotic cells is cultivated under selective conditions to effect recombination. Thus a first set of prokaryotic cells is obtained containing a hybrid molecule with a first and a second recombined DNA sequences due to recombination between the first and the second DNA sequences to be recombined. Also another set of prokaryotic cells is obtained containing a hybrid molecule with a third and a fourth recombined DNA sequences due to recombination between the third and the fourth DNA sequences to be recombined. Then the first, second, third and fourth recombined DNA sequences thus obtained are isolated from their respective host cells. In the next step the first or the second recombined sequence can be inserted into a donor DNA molecule and the third or fourth recombined sequence into a recipient DNA molecule. Both the donor and recipient DNA molecules thus obtained are then introduced into a prokaryotic host cell and subjected another round of recombination. In this way, five, six or more diverging DNA sequences can also be recombined.

In a preferred embodiment of the invention the donor DNA molecule and the recipient DNA molecules are different linear or circular DNA structures. The term "DNA structure" means a DNA molecule, for example a vector such as a plasmid or bacteriophage, which is characterized in that it is present upon introduction into the prokaryotic host cell in the form of an extrachromosomal element. In the context of the present invention an "extrachromosomal element" is therefore a DNA molecule which does not integrate into the chromosome(s) of the prokaryotic host cell.

The donor DNA molecule must be able to hybridize with the recipient DNA molecule so that a hybrid molecule can be formed. Preferably the donor DNA molecule and the recipient DNA molecule do not share homology in general with the exception of the DNA sequences to be recombined.

According to the invention the recipient DNA molecule must be able to autonomously replicate in a prokaryotic host cell upon introduction into that cell. Therefore the recipient DNA molecule must have at least one origin of replication enabling the recipient DNA molecule to replicate independently of the host genetic material. In the context of the present invention an "origin of replication" or "ori" is the region of a DNA molecule that is used by the cellular enzymes to initiate replication of that DNA molecule. At the origin, the two strands of DNA are pulled apart to form a replication bubble, which creates a region of single-stranded DNA on each side of the bubble. The DNA polymerase machinery can then move in and begin to synthesize the new DNA strands, using the old strands as template. Small DNAs including bacterial plasmids or bacteriophages usually have a single origin.

In an embodiment of the invention the recipient DNA molecule is a plasmid, in particular a double-stranded circular DNA molecule. A "plasmid" is an extrachromosomal element which can replicate independently of the host genetic material. In another embodiment of the invention the recipient DNA molecule is a bacteriophage, in particular a phage the DNA of which is present in the prokaryotic cell in the form of an extrachromosomal element.

In a preferred embodiment of the invention the recipient DNA molecule is a plasmid, which can replicate in an *E*. *coli* host cell. Preferably, the recipient DNA molecule is the *E. coli* plasmid pACYC184 or a derivative thereof. Plasmid pACYC184 is Tet^{R} and Cam^{R}.

According to the invention the donor DNA molecule is a DNA molecule which cannot replicate in the prokaryotic host cell but is able to form a hybrid molecule with the recipient DNA. Therefore, any DNA molecule can be used as donor molecule as long as it contains at least a marker sequence and cannot independently replicate within a given prokaryotic host cell. Examples of suitable donor molecule comprise, but are not limited to, linear double-stranded DNAs which can circularize, generated for example by PCR and containing an appropriate marker sequence, plasmids and bacteriophages. In case that a plasmid or a bacteriophage is used as donor DNA molecule this molecule either does not have (a) functional origin(s) of replication at all or has (an) origin(s) of replication which is/are in the prokaryotic host cells used not functional, i.e. not active.

In one embodiment of the invention therefore the donor DNA molecule does not contain an origin of replication. This means, that the donor DNA molecule does not contain any sequences which could function in any prokaryotic host cell as an origin of replication, i.e. sequences to which protein factors involved in the initiation of replication could bind. The absence of an origin of replication can be due to a deletion of those nucleic acid sequences functioning as origin.

In another embodiment of the invention the function of the replication origin of the donor DNA molecule is impaired by one or more mutations abolishing either the function of the origin of replication itself or the function of proteins involved in the replication, in particular those proteins that bind to the nucleic acid sequence of the origin whereby the replication is initiated. For example it is known, that in E. *coli* the key protein for initiation of replication, DnaA, binds to specific sequences, the so-called DnaA-boxes, at the chromosomal origin of replication and melts three AT-rich, 13-mer direct repeats. Additional binding of the DnaA protein to single-stranded 6-mer boxes in the open region is thought to stabilize the open complex (for a review see Jiang et al., PNAS, 100 (2003), 8692-8697). DnaA boxes and AT-rich regions are commonly found at the origin of a variety of prokaryotic replicons and the DnaA protein has been shown to play a key role in the initiation of replication at these origins. Therefore, certain mutations of DnaA boxes and/or AT-rich regions or corresponding sites in the sequence of the replication origin, such as appropriate base substitutions, deletions etc., can prevent the binding of proteins required for initiation of replication and thus inhibit the function of the origin of an extrachromosomal element. In a preferred embodiment of the invention therefore the function of the replication origin of the donor DNA molecule can be impaired by one or more mutations in the nucleic acid sequence of the replication origin of the donor DNA molecule, in particular in the DnaA boxes and/or AT-rich regions of the origin.

Furthermore, it is known that the DnaA protein alone is not sufficient for the formation of an open complex at the origin of plasmids such as RK2, P1, F, pSC101 or R6K. In these cases, efficient formation of an open complex requires binding of the plasmid Rep protein, although in concert with the host DnaA protein and HU or IHF (integrated host factor) protein. The requirement for a plasmid-specified initiation protein for open complex formation is key to the ability of the plasmid to exercise control over the frequency of initiation events at its replication origin (for a review see Jiang et al., PNAS, 100 (2003), 8692-8697). This means, that mutations of plasmid nucleic acid sequences encoding protein factors with essential functions in the plasmid replication can also impair the function of the replication origin of an extrachromosomal element. In another preferred embodiment of the invention therefore the function of the replication origin of the donor DNA molecule can be impaired by one or more mutations in nucleic acid sequences encoding protein factors with essential functions in the replication of the donor DNA molecule.

In still another preferred embodiment of the invention the donor DNA molecule contains an origin of replication which is only active in certain prokaryotic host cells, but not in that prokaryotic cell into which the donor DNA molecule is introduced in order to effect recombination between the two DNA sequences to be recombined. There are numerous reports that an origin of replication that is active in a particular bacterial species does not function in another bacterial species. For example it is known that the replication origin of *Klebsiella pneumoniae* (oriC) is not active in *Caulobacter crescentus, Pseudo-monas putida* or *Rhodobacter sphaeroides* (O'Neill and Bender, J. Bacteriol., 170 (1988), 3774-3777). It is also known that plasmids of *Bacillus subtilis* cannot replicate in *E. coli* cells. In a preferred embodiment therefore the donor DNA molecule and/or its origin of replication are derived from a prokaryotic species other than the prokaryotic species into the cells of which the donor DNA molecule is introduced.

In a particularly preferred embodiment the donor DNA molecule is the *B. subtilis* plasmid pMIX91, which is a derivative of plasmid pIL253 and which can replicate in *B. subtilis,* but not in *E. coli,* and which comprises the *spec^{R}* marker, the *phleo^{R}* marker and the restriction sites *Scal, Ppu*MI and *Eco*O109I for inserting a foreign DNA sequence. In another particularly preferred embodiment the donor DNA molecule is the *B. subtilis* plasmid pMIX101, which is a derivative of plasmid pIL253 and which can replicate in *B. subtilis,* but not in *E. coli,* and which comprises the *tc^{R}* marker sequence and the restriction sites *XhoI* and P*stl* for inserting a foreign DNA sequence.

In another embodiment of the invention the replication origin of the donor DNA molecule is functional only in a certain temperature range, for example at a temperature of less than 45°C. If the donor DNA molecule contains such a temperature sensitive origin it will not be able to replicate under non-permissive conditions, i.e. at a temperature above 45°C, which yet permit growth of the prokaryotic host cell..

In the context of the present invention the term "marker sequences" refers to DNA sequences that are unique in a given prokaryotic cell and that are positioned on either the donor DNA molecule or the recipient molecule, preferably upstream or downstream of a recombination substrate or an already recombined DNA sequence. The presence of a marker sequence on the same molecule of DNA as the recombination substrate or already recombined DNA sequence, preferably in combination with another marker sequence, which can be positioned on the other side of the recombination substrate, allows that recombination substrate or already recombined DNA sequence to be recognized and selected for, whether by molecular or genetic methods.

According to the invention the donor DNA molecule comprises a first marker sequence which allows for the selection of crossovers involving recombination substrates. The first marker sequence, preferably in combination with additional marker sequences present on either the donor DNA molecule or the recipient DNA molecule, also allows further rounds of recombination to be carried out in a iterative fashion.

According to the invention the "first marker sequence" is a protein-encoding DNA sequence, the gene product of which is essential for the prokaryotic host cell to grow and propagate under the selective conditions applied. The first marker sequence is selected from the group consisting of a nutritional marker, an antibiotic resistance marker and a sequence encoding a subunit of an enzyme which functions only, if both or more subunits are expressed in the same cell.

A "nutritional marker" is a marker sequence that encodes a gene product that can compensate an auxotrophy of an organism or cell and thus can confer prototrophy on that auxotrophic organism or cell. In the context of the present invention the term "auxotrophy" means that an organism or cell must be grown in a medium containing an essential nutrient which cannot be synthesized by the auxotrophic organism itself. The gene product of the nutritional marker gene promotes the synthesis of this essential nutrient missing in the auxotrophic cell. Therefore, upon expression of the nutritional marker gene it is not necessary to add this essential nutrient to the medium in which the organism or cell is grown, since the organism or cell has acquired prototrophy.

An "antibiotic resistance marker" is a marker gene wherein the gene product confers upon expression to a cell, in which the expression of the antibiotic marker gene takes place, the ability to grow in the presence of a given antibiotic at a given concentration, whereas a cell without the antibiotic resistance marker cannot.

A "sequence encoding a subunit of an enzyme" can be used as a marker sequence, if a cell cannot synthesize all subunits of an enzyme that are required for the assembly of the complete enzyme structure and thus for obtaining the full activity of the enzyme, and if the presence or absence of the enzymatic activity can be monitored by genetic and/or molecular means. If, for example, the activity of an enzyme is needed for an essential biochemical pathway of the cell, which enables the growth and/or propagation of the cell in a particular environment, and the cell cannot synthesize all components of the complete enzyme structure, then the cell cannot survive in that environment. The "sequence encoding a subunit of an enzyme" used as marker sequence therefore allows upon expression the assembly of the complete enzyme and the survival of the cell.

Preferably, the gene product of the first marker sequence confers resistance to an antibiotic to a cell which is sensitive to that antibiotic. In particular, the first marker-sequence is *spec^{R}* the gene product of which confers to a cell resistance to spectinomycin, *phleo^{R}* the gene product of which confers to a cell resistance to phleomycin or *tc^{R}* the gene product of which confers to a cell resistance to tetracycline.

In another embodiment of the invention the donor DNA molecule contains a second marker sequence. In still another embodiment of the invention the recipient DNA molecule contains a third marker sequence and optionally a fourth marker sequence. That means, in an embodiment of the invention the donor DNA molecule can comprise at least a first and a second marker sequence, optionally even more maker sequences, which can be arranged for example either upstream or downstream of the recombination substrate. In another embodiment the recipient DNA sequence can comprise a third and a fourth marker sequence, optionally even more marker sequences, which can be arranged for example either upstream or downstream of the recombination substrate. These additional marker allow to increase the stringency of selection of the recombined DNA sequences, since the hybrid molecule formed in the second prokaryotic cell and comprising the two different recombined DNA sequences can contain at least four different marker sequences altogether.

According to the invention the "second, third and fourth marker sequences" are protein-coding or non-coding sequences selected from the group consisting of nutritional markers, pigment markers, antibiotic resistance markers, antibiotic sensitivity markers, primer recognition sites, intron/exon boundaries, sequences encoding a particular subunit of an enzyme, promoter sequences, downstream regulated gene sequences and restriction enzyme sites.

A "pigment marker" is a marker gene wherein the gene product is involved in the synthesis of a pigment which upon expression will stain that cell, in which the pigment marker is expressed. A cell without the pigment marker does not synthesize the pigment and is therefore not stained. The pigment marker therefore allows a rapid phenotypical detection of that cell containing the pigment marker.

An "antibiotic sensitivity marker" is a marker gene wherein the gene product destroys upon expression the ability of a cell to grow in the presence of a given antibiotic at a given concentration.

"Primer recognition sites" are annealing sites for site-specific PCR primers, which allow a rapid identification of the respective marker sequences by PCR.

In a preferred embodiment of the invention the second marker sequence of the donor DNA molecule and the third and fourth marker sequences of the recipient DNA molecule are protein-coding sequences, the gene products of which confer resistance to an antibiotic to a cell which is sensitive to that antibiotic. Preferably, the gene product of the third marker sequence confers to a cell resistance to tetracycline and the gene product of the fourth marker sequence confers to a cell resistance to chloramphenicol

In the context of the present invention the terms "DNA sequences to be recombined" and "recombination substrate" mean any two DNA sequences that can be recombined as a result of homologous or non-homologous recombination processes.

Homologous recombination events of several types are characterized by the base pairing of a damaged DNA strand with a homologous partner, where the extent of interaction can involve hundreds of nearly perfectly matched base pairs. In contrast, illegitimate or non-homologous recombination is characterized by the joining of ends of DNA that share no or only a few complementary base pairs. In prokaryotic cells, non-homologous repair and recombination events occur at significantly lower frequencies than homologous recombination events.

In a preferred embodiment of the invention the two recombination substrates, i.e. the first and second DNA sequences to be recombined, are diverging sequences, i.e. sequences, which are not identical but show a certain degree of homology. In the context of the invention the term "homology" denotes the degree of identity existing between the sequences of two nucleic acid molecules, whereas "divergence" denotes the degree of non-identity between the sequences of two nucleic acid molecules. According to the invention the DNA sequences to be recombined differ from each other at two or more positions with respect to their overall alignment. In an embodiment of the invention the overall divergence between the two recombination substrates, relative to their total length, is more than 0,1 %, in particular more than 5 % and preferably more than 25 %. This means, that the DNA sequences to be recombined can differ by more than 30 %, by more than 40 % and even by more than 50 %. In a particularly preferred embodiment of the invention the two DNA sequences to be recombined share at least one or more homologous or identical regions, which, however, can be very short. According to the invention the homologous or identical regions can comprise less than 25 nucleotides, in particular less than 20 or less than 15 nucleotides and even less than 10 nucleotides, for example 4, 5, 6, 7, 8 or 9 nucleotides.

Recombination substrates or DNA sequences to be recombined can have a natural or synthetic origin. Therefore, in one embodiment of the invention the first and the second DNA sequences to be recombined are naturally occurring sequences. Naturally occurring sequences can be isolated from any natural source including viruses, living or dead prokaryotic organisms such as bacteria, living or dead eukaryotic organisms such as fungi, animals, plants and humans, or parts thereof by any appropriate isolation methods or can be synthesized by chemical means. Naturally occurring sequences can also comprise such sequences that after isolation from a natural source were subjected to mutagenesis. In another embodiment of the invention the first and the second DNA sequences to be recombined are artificial sequences that are not found in a natural source. Artificial sequences can be synthesized by any known chemical methods.

In a preferred embodiment of the invention DNA sequences to be recombined are protein-encoding sequences, for example sequences encoding enzymes that can be utilized for the industrial production of natural and non-natural compounds. Enzymes or those compounds produced by the help of enzymes can be used for the production of drugs, cosmetics, foodstuffs, etc. Protein-encoding sequences can also be sequences that encode proteins that have therapeutic applications in the fields of human and animal health. Important classes of medically important proteins include cytokines and growth factors. The recombination of protein coding sequences allows for the generation of new mutated sequences which code for proteins with altered, preferably improved functions and/or newly acquired functions. In this way it is possible, for example, to achieve improvements in the thermostability of a protein, to change the substrate specificity of a protein, to improve its activity, to evolve new catalytic sites and/or to fuse domains from two different enzymes. Protein-encoding DNA sequences to be recombined can include sequences from different species which code for the same or similar proteins that have in their natural context similar or identical functions. Protein-encoding DNA sequences to be recombined can include sequences from the same protein or enzyme family. Protein coding sequences to be recombined can also be sequences which code for proteins with different functions - for example, sequences that code for enzymes which catalyse different steps of a given metabolic pathway. In a preferred embodiment of the invention the first and the second DNA sequences to be recombined are selected from the group of gene sequences of the Oxa superfamily of Beta-lactamases.

In another preferred embodiment of the invention DNA sequences to be recombined are non-coding sequences such as sequences, which, for example, are involved within their natural cellular context in the regulation of the expression of a protein-coding sequence. Examples for non-coding sequences include, but are not limited to, promoter sequences, sequences containing ribosome binding sites, intron sequences, polyadenylation sequences etc. By recombining such non-coding sequences it is possible to evolve mutated sequences, which in a cellular environment result in an altered regulation of a cellular process - for example, an altered expression of a gene.

According to the invention a recombination substrate or DNA sequence to be recombined can of course comprise more than one protein coding sequence and/or more than one non-coding sequence. For example a recombination substrate can comprise one protein-encoding sequence plus one non-coding sequence or a combination of different protein-encoding sequences and different non-coding sequences. In another embodiment of the invention DNA sequences to be recombined therefore can consist of one or more stretches of coding sequences with intervening and/or flanking non-coding sequences. That means, the DNA sequence to be recombined can be for example a gene sequence with regulatory sequences at its 5'-terminus and/or an untranslated 3'-region or an mammalian gene sequence with an exon/intron structure. In still another embodiment of the invention DNA sequences to be recombined can consist of larger continuous DNA stretches that contain more than a single coding sequence, optional with intervening non-coding sequences, such as an operon. DNA sequences to be recombined can be sequences that have already experienced one or more recombination events, for example homologous and/or non-homologous recombination events.

The recombination substrates can comprise-non-mutated wild-type DNA sequences and/or mutated DNA sequences. In a preferred embodiment therefore it is possible to recombine wild-type sequences with already existing mutated sequences in order to evolve new mutated sequences.

According to the invention a prokaryotic cell is used as host cell for introducing the recipient and donor DNA molecules. The terms "prokaryotic cell" and "prokaryotic host cell" include any cell, in which the genome is freely present within the cytoplasm as a circular structure, i.e. a cell, in which the genome is not surrounded by a nuclear membrane. A prokaryotic cell is further characterized in that it is not necessarily dependant on oxygen and its ribosomes are smaller than that of eukaryotic cells. Prokaryotic cells include archaebacteria and eubacteria. In dependence on the composition of the cell wall eubacteria can be divided into gram-positive bacteria, gram-negative bacteria and cyanobacteria.

Therefore, according to the present invention the prokaryotic cell is a cell of an archaebacterium or an eubacterium, whereby in a preferred embodiment of the invention the prokaryotic cell is a gram-negative bacterium, a gram-positive bacterium or an cyanobacterium. Preferably, the gram-negative bacterium is *Escherichla coli,* for example the *E. coli* strain AB1157 or its MutS⁻ mutant, the *E. coli* strain MXP1.

According to the invention it may be preferred to use prokaryotic host cells for the inventive process which have a functional repair system. The mismatch repair (MMR) system is one of the largest contributors to avoidance of mutations due to DNA polymerase errors in replication. However, mismatch repair also promotes genetic stability by ensuring the fidelity of genetic recombination. Whereas in bacteria and also in yeast and mammalian cells, recombination between homeologous DNA substrates containing a few mismatches (< 1%) occurs much less efficiently than between identical sequences, the frequency of recombination (gene conversion and/or crossovers) is dramatically elevated in MMR-defective lines. This means, that the high fidelity of recombination is not only caused by the intrinsic properties of recombination enzymes, but also by the editing of recombination by the mismatch repair system. Thus the mismatch repair machinery has a inhibitory effect on recombination between diverged sequence. In *E. coli* two proteins of the methyl-directed MMR system, namely MutS and MutL, are required for this strong antirecombination activity, whereas the effect of the other MMR system proteins, MutH and UvrD, is less pronounced. In addition to their roles in MMR and homeologous recombination, MMR proteins also play an important role in removing non-homologous DNA during gene conversion.

In another preferred embodiment of the invention, prokaryotic cells that are deficient in the mismatch repair system are used. In the context of the present invention the term "deficient in the mismatch repair system" means that the MMR system of a prokaryotic cell is transiently or permanently impaired. MMR deficiency of a cell or an organism can be achieved by any strategy that transiently or permanently impairs the MMR system including but not limited to a mutation of one or more genes involved in MMR, treatment with an agent like UV light, which results in a global impairment of MMR, treatment with an agent like 2-aminopurine or a heteroduplex containing an excessive amount of-mismatches to transiently saturate and inactivate the MMR system, and inducible expression or repression of one or more genes involved in MMR, for example via regulatable promoters, which would allow for transient inactivation

In a preferred embodiment of the invention the mismatch repair deficiency of the prokaryotic host cell is due to a mutation of at least one of the genes involved in MMR. In a preferred embodiment the prokaryotic cells have a mutated *mutS* gene, a mutated *mutL* gene, a mutated *mutH* gene and/or a mutated *UvrD* gene.

In another embodiment the prokaryotic host cell is impaired or hampered in one or more of the major recombination proteins. It has been shown that a cell impaired for example in the *AddAB* genes shows a higher frequency of homologous and non-homologous recombinationln another embodiment the prokaryotic host cell is overexpressing one of the major recombination proteins such as recA. This protein is involved in homologous recombination by promoting renaturation of single-stranded DNA to form the heteroduplex molecule required for the recombination event and initiates the exchange of DNA strands.

According to the invention the first prokaryotic cell is generated by simultaneously or sequentially introducing the recipient DNA molecule and the donor recipient molecule into the prokaryotic host cell. In one embodiment of the invention, therefore, the first prokaryotic cell can be generated by introducing in a first step the recipient DNA molecule into a particular prokaryotic host cell. After recovery of the prokaryotic host cell harbouring the recipient molecule, the donor DNA molecule is introduced in a second step into the cell harbouring the recipient DNA molecule. In another embodiment of the invention both recipient and donor DNA molecules can be introduced simultaneously into the prokaryotic host cell.

According to the invention the introduction of both the recipient and the donor DNA molecules into the prokaryotic host cell can be effected by any known appropriate method including, but not restricted to, transformation, conjugation, transduction, sexduction, infection and/or electroporation.

In the context of the present invention the term "transformation" means the uptake of an isolated, preferably purified, nucleic acid molecule from the environment by a cell, for example a microbial cell. Cells of some prokaryotic species such as *Bacillus* or *Diplococcus* are naturally competent, whereas cells of other prokaryotic species such as *E. coli* have to be subjected special treatments in order to make them competent, i.e. to induce the transfer of the nucleic acid molecule across the cellular membrane. Several bacteria are known for their ability to exchange DNA through transformation.

"Conjugation" means the plasmid-mediated transfer of a bacterial plasmid from one bacterial cell into another bacterial cell through cell-to-cell-contact. The transfer machinery involved is usually encoded by plasmids or conjugative transposons. Examples of such plasmids are conjugative plasmids or helper plasmids. Conjugative plasmids are self-transmissible plasmids that carry genes that promote cell-to-cell contact (mobilisation genes). They contain the genes for generating conjugation bridges. Once a bridge is made, other plasmids and even chromosomal DNA (conjugative transposons) can also be transferred. Mobilisable plasmids contain mobi lisation genes, but need the "help" of conjugative plasmids to move between cells. Conjugation is one of the major routes for genetic exchange between different phylogenetic groups of prokaryote cells and between prokaryotes and eukaryotes.

"Sexduction" is a process by which genetic material is transferred from a prokaryotic cell with an F-factor or F-plasmid to a cell which does not contain that F-factor (F⁻-cell). The F-factor is usually present in the cytoplasm of a bacterial cell, but can occasionally be incorporated at various sites of the bacterial chromosome, which leads to the formation of Hfr-cells. The integration of the F-factor is reversible, whereby upon an incorrect disintegration of the plasmid so-called substituted F-factors (F') arise that can contain genetic material flanking in the bacterial chromosome the original integration site of the F-factor. The F'-plasmid can be transferred with high frequency into F⁻-cells.

"Transduction" means the transfer of a nucleic acid molecule from one bacterial cell into another bacterial cell by a bacteriophage, which comprises the release of a bacteriophage from one cell and the subsequent infection of the other cell. There are two types of transduction. A spezialized transduction may occur during the lysogenic life cycle of a temperate bacteriophage, whereby genetic material of bacteria can replace a part of the bacteriophage genome. This piece of bacterial DNA replicates as a part of the phage genome and can be transferred by the phage into another recipient cell. In case of a generalized transduction the entire genome of a lytic phage can be replaced by bacterial DNA. When this phage infects another bacterium, it injects the DNA into the recipient where it can be exchanged for a piece of the DNA of the recipient-cell.

"Electroporation" is a process where cells are mixed with nucleic acid molecules and then briefly exposed to pulses of high electrical voltage. The cell membrane of the host cell is rendered penetrable thereby allowing foreign nucleic acids to enter the host cell.

In a particular preferred embodiment of the invention the donor DNA molecule is UV-irradiated prior to introduction into the prokaryotic host cell, since it is known that irradiation leads to an increase of recombination frequencies.

According to the invention the first prokaryotic cell containing the recipient and donor DNA molecules is cultivated under such conditions which force both the formation of a co-integrate or hybrid molecule between the recipient and donor DNA molecules and the recombination, preferably non-homologous recombination, between the two recombination substrates, i.e. conditions which allow for the selection of recombined DNA sequences.

If the first marker sequence of the donor DNA molecule is an antibiotic resistance marker, the first prokaryotic cell containing the recipient and donor DNA molecules is cultivated in the presence of an antibiotic to which the prokaryotic host cell is sensitive and to which the gene product of the first marker sequence confers resistance. In a particularly preferred embodiment the first prokaryotic is cultivated in the presence of spectinomycin if the first marker sequence present on the donor DNA molecule is *spec^{R}* the gene product of which confers to a cell resistance to spectinomycin. In another particularly preferred embodiment the first prokaryotic is cultivated in the presence of phleomycin if the first marker sequence present on the donor DNA molecule is *phleo^{R}* the gene product of which confers to a cell resis-trance to phleomycin. In another particularly preferred embodiment the first prokaryotic is cultivated in the presence of tetracycline if the first marker sequence present on the donor DNA molecule is *tc^{R}* the gene product of which confers to a cell resistance to tetracycline.

If the first marker sequence of the donor DNA molecule is a nutritional marker, the first prokaryotic cell containing the recipient and donor DNA molecules is cultivated in a medium lacking a particular essential nutrient which cannot be synthesized by the host cell itself and which is supplied to the host cell upon introduction of the donor molecule and expression of the first marker gene contained on the donor molecule. Upon expression of the first nutritional marker gene it is not necessary to add this essential nutrient to the medium in which the prokaryotic cell is grown.

If the second marker sequence, the third marker sequence and/or the fourth marker sequence are antibiotic resistance markers then in a preferred embodiment the first prokaryotic cell can be cultivated additionally in the presence of a second, a third and/or a fourth antibiotic to which the gene products of the second marker sequence, the third marker and the fourth marker sequence, respectively, confer resistance. Preferably, the first prokaryotic cell is cultivated not only in the presence of phleomycin or spectinomycin or tetracycline, but in addition also in the presence of chloramphenicol. Most preferably, the first prokaryotic cell is grown and propagated in presence of phleomycin, spectinomycin, chloramphenicol and tetracycline.

After cultivation of the prokaryotic cells under selective conditions second prokaryotic cells are isolated which contain a co-integrate or hybrid molecule formed by the recipient and donor DNA molecules. This co-integrate contains the recombinant DNA sequences. The presence of the co-integrate and/or recombinant DNA sequences can be verified and detected by several means such as restriction profile analysis, PCR amplification and/or sequencing. If, for example the second marker sequence of the donor molecule and the third or fourth marker sequence of the recipient molecule are unique primer recognition sequences then specific fragments of the co-integrate can be PCR-amplified by using appropriate primers recognizing these marker sequences. in order to detect the presence of the respective marker combination. If no co-integrate has formed, i.e. no recombination has occurred, these fragments cannot be detected. If, for example the second marker sequence of the donor molecule and the third or fourth marker sequence of the recipient molecule are unique restriction enzyme cleavage sites then the co-integrate can be subjected to a restriction enzyme analysis in order to detect specific DNA fragments. If no co-integrate has formed, i.e. no recombination has occurred, these fragments cannot be detected.

According to the invention the first and the second recombined DNA sequences contained in the hybrid DNA molecule of the second prokaryotic cell can be isolated and/or analysed and/or selected. The first and second recombined DNA sequences obtained can be isolated for example by PCR amplification or cleavage with appropriate restriction enzymes. An analysis of the first and the second recombined DNA sequences contained in the hybrid DNA molecule can be conducted for example by sequencing methods.

According to the invention the isolated first and second recombined DNA sequences can again be inserted into a donor DNA molecule and a recipient DNA molecule, respectively, and subjected to another round of recombination by using the invention process.

Another aspect of the present invention relates to a process of generating novel proteins, enzymes and non-coding sequences with novel or improved functions and properties, whereby known protein-coding sequences or known non-coding sequences are subjected one or more recombination rounds by using the inventive process for generating and detecting recombinant DNA sequences in a prokaryotic host cell. The present invention also relates to the proteins, enzymes and non-coding sequences, generated by any one of the inventive processes.

The present invention also relates to the *B. subtilis* plasmid pMIX91, which is a derivative of plasmid pIL253 and which can replicate in *B. subtilis,* but not in *E. coli,* and which comprises the *spec^{R}* marker and the *phleo^{R}* marker and the restriction sites *Sca*l*, Ppu*MI and *Eco*O109I for inserting a foreign DNA sequence.

The present invention also relates to the *B. subtilis* plasmid p-MIX101, which is a derivative of plasmid pIL253 and which can replicate in *B. subtilis,* but not in *E. coli,* and which comprises the *tc^{R}* marker sequence and the restriction sites *Xhol* and *PstI* for inserting a foreign DNA sequence.

The present invention also relates to *B. subtilis* strain DSM4393, containing the *B. subtilis* plasmid pMIX91 (deposited at the DSMZ, Deutsche Sammlung für Mikroorganismen und Zellkulturen GmbH, Braunschweig, Germany on 21^{st} February 2005, SB202pMIX91) and to *B*. *subtilis* strain 1A423, containing the *B. subtilis* plasmid pMIX101 (deposited at the DSMZ, Deutsche Sammlung für Mikroorganismen und Zellkulturen GmbH, Braunschweig, Germany on 21^{st} February 2005, 1A423pMIX101).

Another aspect of the present invention relates to the use of the *B. subtilis* plasmid pMIX91 and pMIX101, respectively, as donor DNA molecules in the inventive process, i.e. the use of plasmid pMIX91 or pMIX101 for generating and/or detecting recombinant DNA sequences in a prokaryotic host cell, preferably in an *E. coli* cell.

Another aspect of the present invention relates to the use of the *E. coli* plasmid pACYC184 or pMIX100 or derivatives thereof as recipient DNA molecule in the inventive process, i.e. the use of plasmid pACYC184 or pMIX100 for generating and/or detecting recombinant DNA sequences in a prokaryotic host cell, preferably in an *E. coli* cell.

Another aspect of the present invention relates to a kit which can be used for conducting the inventive process for generating and detecting recombined DNA sequences in a prokaryotic host cell. In a first embodiment the kit comprises at least a first container which comprises cells of the *E. coli* strain AB1157 as prokaryotic host cell, a second container which comprises cells of the *E. coli* strain AB1157 containing the *E. coli* plasmid pACYC184 or the *E. coli* plasmid pMIX100, which can be used as recipient DNA molecule, and a third container comprising cells of the *B. subtilis* strain DSM4393, containing the *B. subtilis* plasmid pMIX91, or cells of the *B. subtilis* strain 1A423, containing the *B. subtilis* plasmid pMIX101, which can be used as donor DNA molecule. In a second embodiment of the invention the kit comprises at least a first container which comprises cells of the *E. coli* strain MXP1, which is a MutS⁻ mutant of strain AB1157, as prokaryotic host cell, a second container which comprises cells of the *E. coli* strain AB1157 containing plasmid pACYC184 or pMIX100 and a third container comprising cells of the *B. subtilis* strain DSM4393, containing the plasmid pMIX91, or cells of the *B. subtilis* strain 1A423, containing the plasmid pMIX101. In still another embodiment the kit comprises at least a first container which comprises either cells of the *E. coli* strain AB1157 or the *E. coli* strain MXP1, a second container comprising DNA of the E. coli plasmid pACYC184 or pMIX100 and a third container comprising DNA of the *B. subtilis* plasmid pMIX91 or the *B. subtilis* plasmid pMIX101.

Another aspect of the present invention relates to a process for producing a hybrid gene and/or a protein encoded by a hybrid gene in a prokaryotic cell. The process for producing a hybrid gene and/or the encoded protein comprises the step of conducting the inventive process for generating and detecting recombinant DNA sequences, whereby the hybrid gene and/or the protein encoded by the hybrid gene is produced in the prokaryotic cell. After expression the hybrid gene and/or the encoded protein is selected in the prokaryotic cell and/or isolated therefrom.

The present invention also relates to a hybrid gene that is obtainable by the inventive process for producing a hybrid gene or the inventive process for generating and detecting recombinant DNA sequences.

The present invention also relates to a protein, which is encoded by a hybrid gene, that is obtainable by the inventive process for producing a hybrid gene or the inventive process for generating and detecting recombinant DNA sequences, and/or which is obtainable by the inventive process for producing a protein encoded by a hybrid gene.

The present invention is illustrated by the following sequence listing, figures and example.

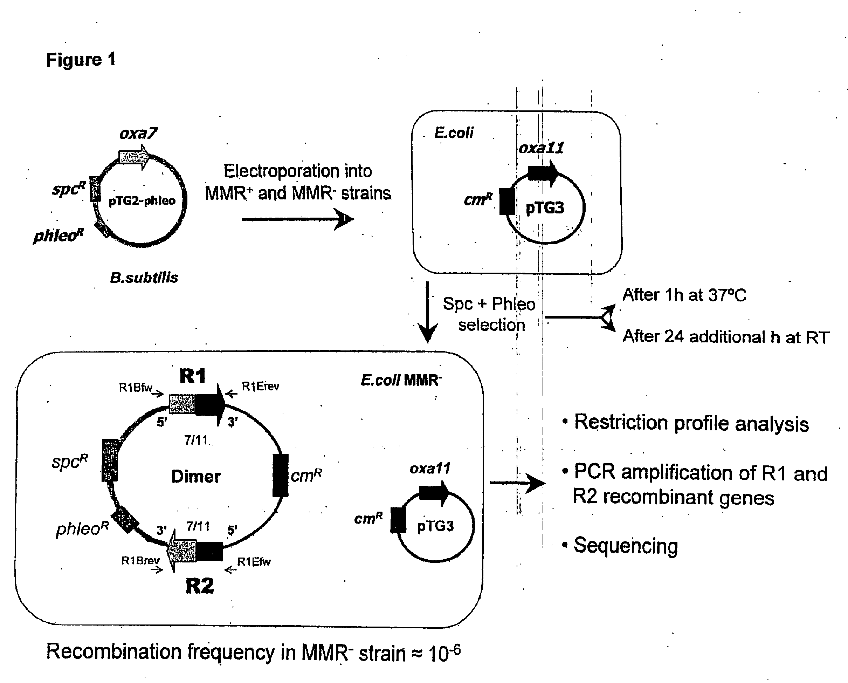

Figure 1 illustrates schematically the inventive process for generating and/or detecting recombination between two DNA sequences. The first sequence, the *oxa7* gene, is present on the *B. subtilis* plasmid pTG2-phleo as donor DNA molecule. pTG2-phleo carries the *spec^{R}* and *phleo^{R}* markers which confer resistance to spectinomycin and phleomycin. By electroporation pTG2-phleo is introduced into a *E. coli* host cell containing the plasmid pTG3 as recipient DNA molecule. pTG3 contains the second DNA sequence, the *oxa11* gene, and the *cm*^{R} marker conferring resistance to chloramphenicol. After introduction of pTG2-phleo the cell is cultivated in presence of spectinomycin and phleomycin which forces the formation of a co-integrate between the two plasmids and concomitantly recombination between the genes. Therefore, upon cultivation an *E. coli* cell is obtained containing a dimeric plasmid containing the newly recombined DNA sequences R1 and R2. The recombined DNA sequences can be analyses by restriction profile analysis, PCR amplification of R1 and R2 recombinant genes and/or sequencing of R1 and R2.

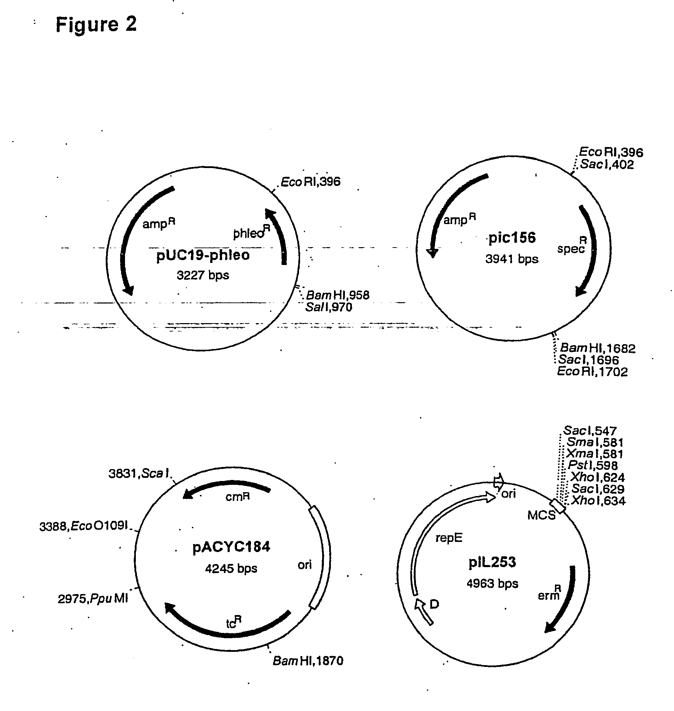

Figure 2 shows physical maps of the plasmids pUC19-phleo, pic156, pACYC184 and pIL253 used to construct suitable plasmids for conducting the inventive process.

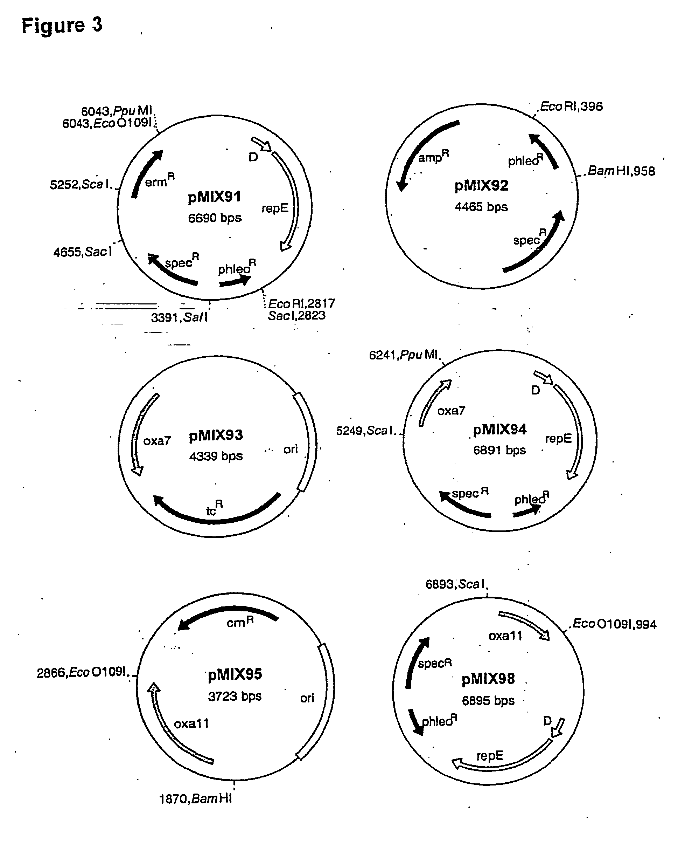

Figure 3 shows physical maps of plasmids which were constructed for conducting the inventive process. The plasmids pMIX96 and pMIX 97 are not depicted since they are as pMIX95 but with *oxa5* and *oxa1,* respectively, instead of *oxa11.* pMIX99 is as pMIX98, but with *oxa1* instead of *oxa11.*

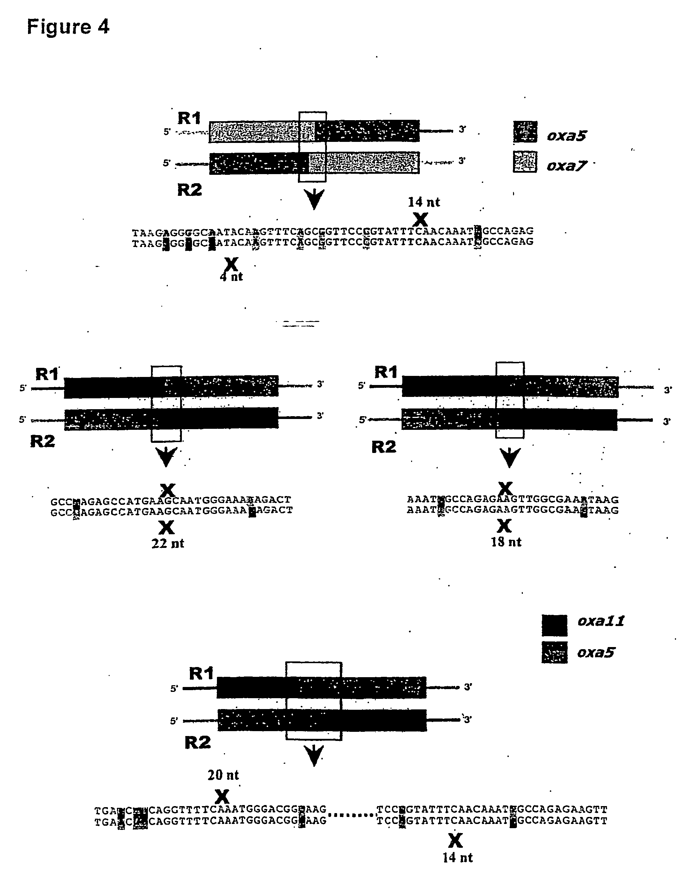

Figure 4 shows the structure of genes obtained by the inventive in vivo recombination process between 22%-divergent *oxa* genes. Stretches of sequence identity in which crossovers took place are detailed.

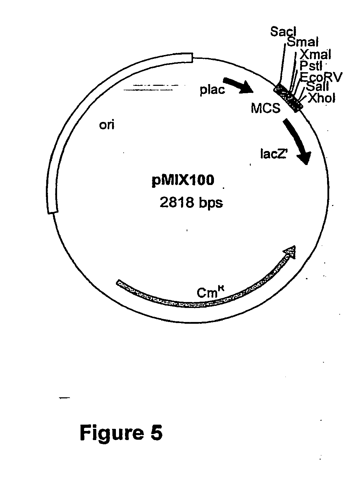

Figure 5 shows the structure of the *E. coli* plasmid pMIX100 which can be used as recipient DNA molecule in an *E. coli* host cell. PMIX100 carries the origin of replication from plasmid pACYC184, as well as the chloramphenicol-resistance gene therefrom. PMIX100 also carries the gene *lacZ* from pBluescript SK+.

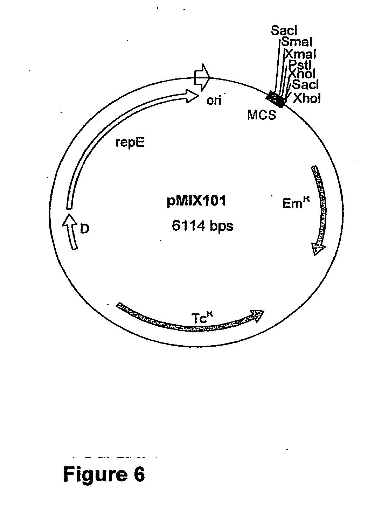

Figure 6 shows the structure of the *B. subtilis* plasmid pMIX101 which is a derivative of the *B. subtilis* plasmid pIL253 and which can be used as donor DNA molecule in an *E. coli* host cell. pMIX101 carries the marker *Erm^{R}* which confers resistance to erythromycin and *Tc^{R}* which confers resistance to tetracycline. The tetracycline-resistance gene was amplified from plasmid pACYC184.

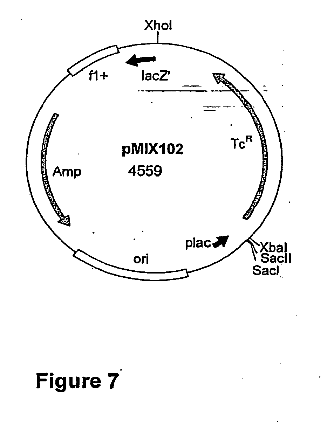

Figure 7 shows the structure of the transformation efficiency control plasmid pMIX102. pMIX102, a derivative of pBluescript SK+, contains the *Tc^{R}* gene amplified from plasmid pACYC184. In pMIX102 the *Tc^{R}* gene is driven by the plac promotor

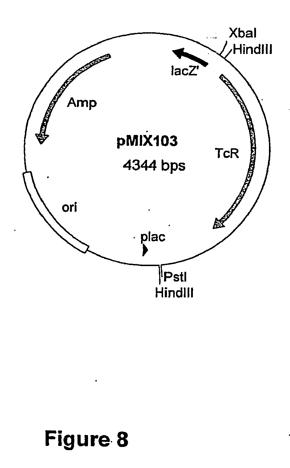

Figure 8 shows the structure of the transformation efficiency control plasmid pMIX103. pMIX103, a derivative of pBluescript SK+, contains the *Tc^{R}* gene amplified from plasmid pACYC184. In pMIX103 the *Tc^{R}* gene is cloned in the opposite direction than plac. Therefore, the gene is expressed from its own promoter.

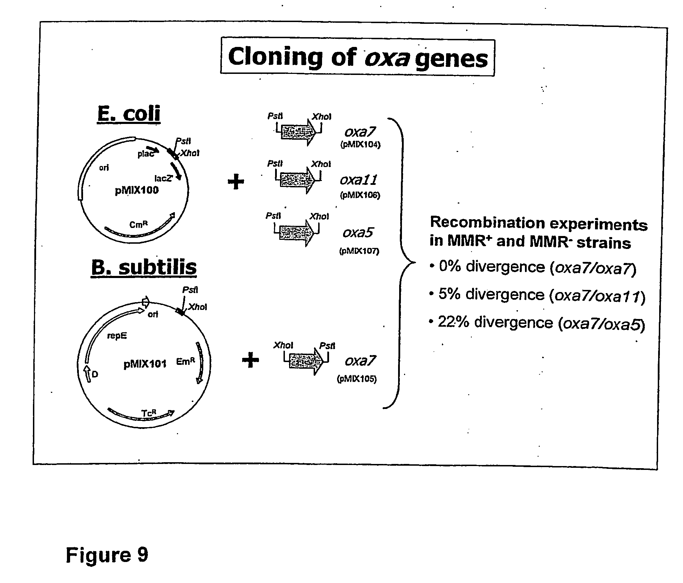

Figure 9 illustrates schematically the strategy for cloning the *oxa7, oxa11* and *oxa5* genes into the *E. coli* plasmid pMIX100 and into the *B. subtilis* plasmid pMIX101. For cloning *oxa7, oxa11* and into pMIX100 the genes were amplified using primers containing either *Pst*I or *Xho*l at their 5' ends. After digestion with those enzymes, the amplified DNA fragments were independently ligated with pMIX100, which was previously cut with *Pst*I + *Xho*l. Competent cells of *E. coli* DHB10 were electroporated with the ligation mixtures, and colonies harboring positive clones were phenotypically selected by chloramphenicol-resistance/white color on LB plates containing Cm (30 µg/ml) + X-Gal (80 µg/ml) + IPTG (0.5 mM). Plasmid DNAs were obtained and analysed by restriction mapping. Results confirmed that pMIX104 carries *oxa7*, pMIX106 carries *oxa11* and pMIX107 carries *oxa5.* For cloning into the *B. subtilis* plasmid pMIX101 *oxa7* was obtained as a 0.9-kb fragment from pMIX104 by restriction with *Pst*I and *Xho*l and ligated with pMIX101, which was previously cut with the same enzymes. After transformation of *B. subtilis* 1A423 competent cells with the ligation product, cells were selected in LB containing 0.5 µg/ml of erythromycin (Erm).

SEQ ID No. 1 and 2 show the sequences of the primers OLG1 and OLG2, respectively, for the amplification of *oxa7* and the introduction of *Sca*I and *Ppu*MI restriction sites at the 5' and 3' ends, respectively.

SEQ ID No. 3 and 4 show the sequences of the primers OLG3 and OLG4, respectively, for the amplification of *oxa11* and the introduction of *Bam*HI and *Eco*O109I restriction sites at the 5' and 3' ends, respectively.

SEQ ID No. 5 and 6 show the sequences of the primers OLG5 and OLG6, respectively, for the amplification of *oxa5* and the introduction of *Bam*HI and *Eco*O109I restriction sites at the 5' and 3' ends, respectively.

SEQ ID No. 7 and 8 show the sequences of the primers OLG7 and OLG8, respectively, for the amplification of *oxa1* and the introduction of *Bam*HI and *Eco*O109I restriction sites at the 5' and 3' ends, respectively.

SEQ ID No. 9 and 10 show the sequences of the primers OLG9 and OLG110, respectively, for the amplification of *oxa11* and the introduction of *Sca*I and *Eco*O109I restriction sites at the 5' and 3' ends, respectively.

SEQ ID No. 11 shows the sequence of primer OLG11, which was used together with primer OLG8 (SEQ ID No.8) for the amplification of *oxa1* and the introduction of *Sca*I and *Eco*O109I restriction sites at the 5' and 3' ends, respectively.

SEQ ID No. 12 and 13 show the sequences of the primers OLG12 and OLG13, respectively, for the amplification of the recombinant gene R1 contained on the hybrid plasmid pMIX93.

SEQ ID No. 14 shows the sequence of primer OLG14, which was used together with primer OLG12 (SEQ ID No. 12) for the amplification of the recombinant gene R1 contained on one of the hybrid plasmids pMIX95, pMIX96 and pMIX97.

SEQ ID No. 15 shows the sequence of primer OLG15, which was used together with primer OLG17 (SEQ ID No. 17) for the amplification of the recombinant gene R2 contained on the hybrid plasmid pMIX93.

SEQ ID No. 16 shows the sequence of primer OLG16, which was used together which was used together with primer OLG17 (SEQ ID No. 17) for the amplification of the recombinant gene R2 contained on one of the hybrid plasmids pMIX95, pMIX96 and pMIX97.

SEQ ID No. 17 shows the sequence of primer OLG17.

### Example 1

### In vivo recombination of heterologous genes using a double plasmid system (E. coli/B. subtilis) whereby recombinant DNA sequences are selected by resistance to spectinomycin and/or phleomycin

### 1. Material and methods

### 1.1 Bacterial strains and plasmids

Bacterial strains and plasmids used in this example are shown in Table 1 and Table 2, respectively.

**Table 1: Bacterial strains**

| **Strains** | **Genotype** | **Reference or source** |
|---|---|---|
| *E. coli* AB 1157 Nal^{R} hsd⁻ | *thr1 leu6 proA2 his4, thil argE3 lacY1 galK2 ara14 xyl15 mtl1 tsx33 str31 supE44thr*⁺ *hsdR⁻B nal^{R}* | M. Radman strain collection |
| *E. coli* MIXP1 | As AB1157 *Nal^{R} R⁻* but *mutS::Tn5 (kan^{R})* | mutS allele from M. Radman strain collection |
| *E. coli DH5*α | *SupE44* Δ*lac*U169 (φ80/*acZ* ΔM15) *hsdR17 recA*1 *endA*1 *gyrA*96 *thi*-1 *rel A*1 | (8) |
| *B. subtilis* DSM4393 | *aroB2 trpC2 his6* | German Strains collection DSMZ (Deutsche Sammlung von Mikroorgansimen und Zellkulturen GmbH) |

**Table 2: Plasmids**

| **Plasmids** | **Selection** | **Reference or source** |
|---|---|---|
| pACYC184 | Tet^{R} Cam^{R} | New England Biolabs (1) |
| pIL253 | Erm^{R} | (6) |
| pic156 | Amp^{R} Spec^{R} | (7) |
| pUC19-Phleo | Amp^{R} Phleo^{R} | (2) |

### 1.2 Growth conditions and culture media

Both *E*. *coli* and *B. subtilis* strains were cultured at 37° C in LB medium (Difco Laboratories, Detroit, USA). When medium was used in plates (LBA), 15 g of agar (Difco) was added per liter. When required medium was supplemented with antibiotics the final concentrations were as follows: tetracycline (Sigma-Aldrich Chimie, St. Quentin Fallavier, France), 12.5 µg/ml; chloramphenicol (Sigma-Aldrich Chimie), 30 µg/ml; ampicillin (Sigma-Aldrich Chimie), 100 µg/ml; erythromycin (Sigma-Aldrich Chimie), 0.5 µg/ml; spectinomycin (Sigma-Aldrich Chimie), 75 µg/ml; phleomycin (Euromedex, Strasbourg, France), 2 µg/ml. When LBA was supplemented with spectinomycin plus phleomycin the final concentrations were 60 and 1 µg/ml, respectively.

### 1.3 Manipulation of DNA and microorgansims

### Transduction

*E*. *coli* MIXP1 strain was constructed using P1-mediated transduction (3).

### Transformation

Plasmids were introduced in *E*. *coli* strains by electrotransformation using an Eppendorf Electroporator 2510 (Eppendorf AG, Hamburg, Germany) and according to the supplier's instructions.

Competent cells of *B*. *subtilis* were prepared and transformed as described by Yasbin et al. (9)

### DNA manipulations

Established protocols were followed for molecular biology techniques (4). Enzymes for DNA manipulations were purchased from New England Biolabs (Beverly, MA, USA), MBI Fermentas (Vilnius, Lithuania), Promega (Madison, Wis.) or Stratagene (La Jolla, CA, USA) and used as recommended by the manufacturers. When necessary, DNA digested with restriction endonucleases was purified from agarose gels using the NuceloSpin Extract Kit (Machery-Nagel).

Plasmid DNA was isolated from E. coli using the NucleoSpin kit (Machery-Nagel GmbH & Co., Düren, Germany) according to the instructions of the manufacturer. To isolate plasmid DNA from B. subtilis the same kit was used but a first lysis step was performed by incubating cells with 2 mg ml⁻¹ of lysozime 30 min at 37°C.

Primers used were synthesized by Proligo France SAS (Paris, France).

Nucleotide sequences were determined in both directions by Genome Express (Meylan, France). Sequences were analysed with the Infobiogen package (Genopole d'Evry, Evry, France). The ClustalW program was used for sequence comparisons.

### PCR amplifications

PCR reactions were performed using a Mastercycler Gradient (Eppendorf AG, Hamburg, Germany). Reactions were carried out in 50-µl volume using the high-fidelity Herculase Enhanced DNA polymerase (Strategene) under the following conditions: 96°C for 3 min, 35 cycles of 96°C for 30 s, annealing temperature for 30 s, 72°C for 1 min, and a final elongation step at 72°C for 10 min. Annealing temperature was determined by subtracting 5°C to the lowest Tm of primers used. When necessary, PCR products were purified using the NucleonSpin Extract kit (Machery-Nagel). Amplification products were analysed by electrophoresis in 0.7% agarose gels (Sigma).

### 2. General strategy

This experiment was conducted in order to generate new molecules exhibiting advantageous properties by in vivo recombination of two parental genes, which share different degrees of sequence identity. The strategy employed is as follows:

Genes that will be recombined are carried by two different plasmids showing no homology in their nucleotide sequences. The first one is an *E*. *coli*-replicative plasmid conferring resistance to either chloramphenicol (Cm) or tetracycline (Tc). It is based on the standard cloning vector pACYC184, a low-copy number plasmid obtained from New England Biolabs.

The second one is a *Bacillus subtilis* plasmid derived from pIL253 (Simon and Chopin, 1988). It is not able to replicate in E. coli and carriers two antibiotic resistance markers for spectinomycin (Spc) and phleomycin (Phleo), respectively.

To recombine pairs of heterologous genes carried by these two vectors, the *B*. *subtilis* plasmid is introduced by electroporation into *E*. *coli* strains harbouring the replicative plasmid. This is shown schematically in Figure 1. Such strains are either proficient (+) or deficient (-) for the mismatch repair system (MMR), which controls both mutagenesis and recombination.

After electroporation, transformants are selected with antibiotics for which the *B*. *subtilis* plasmid confers resistance (Spc and Phleo). Such selective pressure force recombination between heterologous genes, since only cells harbouring a co-integrate formed between the *B*. *subtilis* and the *E*. *coli* plasmids can grow under those conditions. Hybrid plasmid confers resistance to Spc and Phleo and replicates from the *E*. *coli* origin, since that of *B*. *subtilis* is not functional. Moreover, it carries the two recombinant genes R1 and R2.

The first step was the cloning in the *E*. *coli* and *B*. *subtilis* vectors of genes initially chosen as target to evaluate recombination efficiency.

Recombination experiments between *oxa* genes were performed in both wild type and mismatch repair deficient strains. Experiments were done between pairs of either identical or divergent genes.

Plasmid DNAs were UV-irradiated prior to electroporation, since it was shown that irradiation leads to a 10-fold increase of recombination frequencies.

Recombination was also performed in strains temporarily rendered mutator by treatment with 2-aminopurine. 2-aminopurine is an adenine analog that is incorporated into DNA during growth of bacteria and saturates the MMR system. Thus, a transient mutator phenotype is generated, since after removing of 2-aminopurine a wild type status is recovered. This temporal control of the mismatch repair activity provides a stable background to strains used in recombination avoiding the accumulation of mutations in their genomes.

### 3. Results

### Construction of the B. subtilis vector

In order to construct a *B*. *subtilis* vector to carry target genes for recombination, two gene markers conferring resistance to antibiotics spectinomycin (*spec*^{R}) and phleomycin (*phleo*^{R}), respectively, were cloned in the plasmid pIL253 following a two steps cloning strategy.

First, the *spec*^{R} gene was obtained as a *Sacl* fragment of 1294 bps in length from the plasmid pic156. The fragment was purified and then ligated to *Sac*l-digested pIL253. *B. subtilis* DS4393 competent cells were transformed with the ligation mixture and transformants were selected on LBA plates containing 75 µg ml⁻¹ of spectinomycin. Restriction analyses of plasmid DNA obtained from transformants confirmed that they harboured pILF253-derivatives carrying spec^{R} gene.

In a second step, the *phleo*^{R} gene was cloned into pIL253-spec. Plasmid pUC19-phleo was digested with *Eco*RI and *Sal*I restriction enzymes and the fragment of 574 bps in length corresponding to *phleo*^{R} was gel-purified and ligated to pIL253-spec, previously digested with the same two enzymes. *B*. *subtilis* DSM4393 competent cells were transformed with the ligation mixture and transformants were then selected on LBA plates containing 60 µg ml⁻¹ of phleomycin. Restriction analyses of plasmid DNA obtained from transformats demonstrated that they harboured the expected 6.69-kb plasmid, carrying both *spec^{R}* and *phleo^{R}* genes. Plasmid was named pMIX91 (see figure 3).

### Construction of transformation efficiency control vector

A vector was constructed to be used as a control of transformation efficiency of *E*. *coli* strains under spectinomycin and phleomycin selection in recombination experiments. The vector was constructed as follows: the spectinomycin resistance gene (*spec^{R}*) was obtained as a fragment of 1.25 kb after digestion of pic156 with *Bam*H1 and *Eco*RI. It was cloned into the corresponding sites of pUC-phleo, next to the phleomycin resistance gene (*phleo^{R}*). After electroporation of *E. coli* DH10B component cells with the ligation mixture, spectinomy-cin and phleomycin resistant colonies were selected on LBA plates containing both antibiotics at final concentrations of 60 µg/ml and 1 µg/ml, respectively. Restriction analyses of plasmid DNA isolated from transformants showed that they corresponded with the expected construct of 4.46 kb in length, which was named pMIX92 (see figure 3).

### Cloning of genes encoding for β-lactamases into E. coli- and B. subtilis vectors

Four genes encoding for β-lactamases were chosen as target to evaluate recombination efficiencies in both wild-type and MutS⁻ mutant E. coli strains. Such genes, *oxa7* (GenBank accession number X75562), *oxa11* (GenBank accession number Z22590), *oxa5* (GenBank accession number X58272) and *oxa1* (GenBank accession number J02967) display different degrees of divergence in their nucleotide sequences. The sequence of *oxa1* and the sequences of *oxa5*, *oxa7* and *oxa11*, respectively, diverge by 40%. The sequence of *oxa5* and the sequences of *oxa* 7 and *oxa11*, respectively, diverge by 22%. The sequence of *oxa7* and the sequence of *oxa11* diverge by 5%.

The four *oxa* genes were cloned into the *E coli* plasmid pACYC184, whereas *oxa7*, *oxa11 and oxa1* were cloned into the *B*. *subtilis* plasmid pMIX91 as well. The cloning of the genes was done as follows: *oxa7* was amplified by PCR with primers designed to introduce *Sca*I and *Ppu*MI sites at the 5' and 3' ends, respectively, of the amplified DNA (primers OLG1 with SEQ ID No. 1 and OLG2 with SEQ ID No. 2). The PCR product was digested with those restriction enzymes and the resulting fragment of 991 bps in length was ligated to pMIX91, previously cut with the same enzymes. *B. subtilis* DSM4393 competent cells were transformed with the ligation mixture and transformants selection was done on LBA plates containing 75 µg ml⁻¹ of spectinomycin. Restriction analyses of plasmid DNA obtained from tranformants demonstrated that they harboured the expected 6.89 kb plasmid pMIX94 carrying *oxa7* (see figure 3).

To clone *oxa7* into the *E. coli* vector pACYC184, the PCR product described above, as well pACYC184, were digested with *Ppu*MI and Scal. Blunt ends were generated from digested DNAs by using the Klenow fragment of DNA polymerase I, and ligation between them was done. *E*. *coli* DH10B competent cells were electroporated with the ligation mixture, and transformants were selected on LBA plates containing 12.5 µg ml⁻¹ of tetracycline. Restriction analyses of plasmid DNA isolated from transformants showed that they corresponded with the expected construct of 4.33 kb in length, which was named pMIX93 (see figure 3).

To clone *oxa11*, *oxa5* and *oxa1* into pACYC184, genes were amplified by PCR using primers designed to introduce *Bam*HI and *Eco*O1091 sites at the 5' and 3' ends, respectively, of the amplified DNA. OLG3 (SEQ ID No. 3)/OLG4 (SEQ ID No. 4), OLG5 (SEQ ID No. 5)/OLG6 (SEQ ID No. 6) and OLG7 (SEQ ID No. 7)/OLG8 (SEQ ID No. 8) primers pairs were used to amplify *oxa11*, *oxa5* and *oxa1* respectively. The PCR products were digested with *Bam*HI and *Eco*O1091 and the resulting fragment of 997 bps (*oxa11*) and 830 bps (*oxa5*) and 936 bps (*oxa1*) were independently ligated to pACYC184, previously cut with the same enzymes. *E. coli* DH10B competent cells were electroporated with the ligaton mixtures, and transformants were selected on LBA plates containing 30 µg ml⁻¹ of chloramphenicol. Restriction analyses of plasmid DNA isolated from tranformants showed that they corresponded the expected 6.89 kb plasmids pMIX95 (3.72 kb), pMIX96 (3.55 kb) and pMIX97 (3.66 kb) (see figure 3).

To clone *oxa11* and *oxa1* into pMIX91, genes were amplified by PCR using primers designed to introduce *Sca*I and *Eco*O109I sites at the 5' and 3' ends, respectively, of the amplified DNA. OLG9 (SEQ ID No. 9)/OLG10 (SEQ ID No. 10), and OL11 (SEQ ID No.11)/OLG8 primers pairs were used to amplify *oxa11* and *oxa1* respectively. The PCR products were digested with *Sca*I and *Eco*O109I and the resulting fragment of 995 bps (*oxa11*) and 934 bps (*oxa1*) were independently ligated to pMIX91, previously cut with the same enzymes. *B*. *subtilis* DSM4393 competent cells were transformed with the ligation mixture and transformants selection was done on LBA plates containing 75 µg ml⁻¹ of spectinomycin. Restriction analyses of plasmid DNA obtained from transformants demonstrated that they correspond to the expected plasmids pMIX98 (6.89kb) and pMIX99 (6,83 kb). See figure 3.

### In vivo recombination of oxa genes in wt compared to MutS⁻ mutant E. coli

In a first step, competent cells of *E*. *coli* AB1157 and its MutS⁻ mutant, *E. coli* MXP1, were independently transformed by electroporation with pACYC184-derivative plasmids carrying *oxa* genes, pMIX93, pMIX95, pMIX96 or MIX97. Transformants were selected on the basis of their resistance to either tetracycline or chloramphenicol. The presence of the suitable plasmids was subsequently confirmed by restriction and/or PCR analyses.

In a second step, competent cells of both wild-type and MutS⁻ strains harbouring replicative plasmids were prepared from selective media containing either tetracycline or chloramphenicol. Afterwards such competent cells were independently transformed by electroporation with the *B*. *subtilis*-plasmids carrying *oxa* genes, pMIX94, pMIX98 or pMIX99.

After electroporation, tranformants were selected on LBA plates containing antibiotics for which the *B*. *subtilis* plasmid confers resistance: spectinomycin and phleomycin, at final concentrations of 60 µg ml⁻¹ and 1 µg ml⁻¹, respectively. Such selective pressure force recombination between oxa genes, since only cells harbouring a hybrid plasmid formed between the B. subitlis and the *E*. *coli* plasmids can grow in those conditions. Plates were incubated overnight at 37° C, and afterwards plasmid DNA of transformants were analyzed by digestion with restriction enzymes in order to confirm that they harboured hybrid plasmids (of about 10.5 kb in length) carrying two recombinant genes, R1 and R2. In some cases, recombinant genes were amplified by PCR and sequenced. If the resident plasmid was pMIX93 R1 was amplified with OLG12 (SEQ ID No. 12)/OLG13 (SEQ ID No. 13) and R2 with OLG15 (SEQ ID No. 15)/OLG17 (SEQ ID No.17); if the resident plasmid was pMIX95, pMIX96 or pMIX97 R1 was amplified with OLG12/OLG14 (SEQ ID No. 14) and R2 with OLG16 SEQ ID No. 16)/OLG17.

In recombination experiments the *B. subtilis* plasmid pMIX91, was used as a negative control, whereas the *E*. *coli* plasmid pMIX92 was used as control of transformation efficiency under spectinomycin and phleomycin selection. pMIX92 can replicate in strains harbouring pACYC184-derivative plasmids since their origins of replication (ColE1 and p15, respectively) are compatible. Plasmid DNAs were UV-irradiated prior to electroporation (200J/m²), since it was shown that irradiation leads to a 10-fold increase of recombination frequencies.

Recombination frequencies were calculated by dividing the transformation efficiency obtained with *B. subtilis* plasmids per the transformation efficiency obtained with control vector pMIX92. Transformation efficiencies were in turn calculated as number of colony forming units (cfu) obtained per µg of DNA on previously described conditions.

Results obtained are summarized in table 3. In the wild-type strain, recombinants were obtained in experiments using either identical or 5% divergent *oxa* genes, whereas in the MutS⁻ mutant, recombination also happened between 22% divergent genes.

**Table 3: In vivo recombination frequencies obtained between oxa genes in both wild-type and MutS⁻ E. coli strains.**

| **Genetic divergence** **(%)** | **Recombination frequencies** | |
|---|---|---|
| | **wt strain** | **MutS⁻ strain** |
| 0 | 10⁻⁴ | 10⁻⁴ |
| 5 | 10⁻⁸ | 10⁻⁵ |
| 22 | ---- | 10⁻⁷ |
| 40 | ---- | ---- |

### In vivo recombination of oxa genes in wt compared to 2-aminopurine (2-AP) treated E.coli

In a first step, competent cells of *E*. *coli* AB1157 were transformed by electroporation with pACYC184-derivative plasmids carrying *oxa 11* gene, pMIX95. Transformants were selected on the basis of their resistance. The presence of the suitable plasmids was confirmed by restriction and/or PCR analyses.

In a second step. competent cells of this strain were prepared in presence of 200 µ g/ ml of 2-AP and independently electroporated with the *B*. *subtilis* plasmids pMIX94 carrying *oxa7* or pMIX98 carrying *oxa11*.

The *B*. *subtilis* plasmid pMIX91 was used as a negative control. whereas the *E*. *coli* plasmid pMIX92, carrying Spc^{R} and Phleo^{R} markers, was used as control of transformation efficiency. Plasmid DNAs were UV-irradiated before electroporation in order to increase recombination frequency.

Results are summarized in the table 4. In the wild-type strain, recombinants were obtained in experiments using identical *oxa* genes, whereas in the 2-AP treated strain, recombination also happened between 5% divergent genes.

**Table 4: In vivo recombination frequencies obtained between oxa genes in both wild-type and 2-AP treated E. coli strains.**

| **Genetic divergence** **(%)** | **Recombination frequencies** | |
|---|---|---|
| | **wt strain** | **2-AP treated strain** |
| 0 | 10⁻⁴ | 10⁻⁵ |
| 5 | --- | 10⁻⁶ |

It was noteworthy that recombination occurred between 22% divergent genes, in which the longest stretch of sequence identity was 22 nucleotides (oxa5/oxa11) and 18 nucleotides (oxa5/oxa7), respectively (see figures 5 and 6). It has been reported that recombination becomes inefficient below a minimal length of homology, known as MEPS (minimal efficient processing segment). Length of MEPS varies dependent on the recombination pathway but it has been described to range from 23 to 90 base pairs (5).

Sequence analyses of genes R1 and R2 carried by 54 hybrid plasmids obtained in experiments involving either 5% or 22% divergent genes, showed that 46 of those hybrid plasmids were different to each other. This result indicates that they corresponded to different events of recombination, and consequently, that a high degree of genetic diversity was generated by in vivo recombination.

Most of recombinant genes were generated by non-reciprocal single crossovers in different stretches of sequence identity ranging from 4 to 101 nucleotides. In some cases, multiple crossovers were observed, producing either R1 or R2 mosaic genes. Recombinant genes obtained between *oxa7*/*oxa5* and *oxa11*/*oxa5* are depicted in figure 4.

The comparison of DNA and deduced amino acid sequences of recombinant genes revealed as well that 53% of them corresponded to new *oxa* genes (see table 4). Since no frameshifts or stop codons were generated during recombination, they could putatively encode for 38 new functional β-lactamases.

**Table 4: Comparison of nucleotide and deduced amino acid sequences of recombinant oxa genes obtained by in vivo recombination**

| **Sequence** **comparisons** | **New genes** | **New proteins** |
|---|---|---|
| **R1** | 33/54 | 21/54 |
| **R2** | 25/54 | 17/54 |
| **Total** | 58/108 (53%) | 38/108 (35%) |

### Example 2

### In vivo recombination of heterologous genes a double plasmid system (Escherichia coli/Bacillus subtilis) whereby recombinant DNA sequences are selected by resistance to tetracycline

Another double plasmid system was designed to verify the results obtained in example 1 and facilitate cloning and recombination of genes. This system is based on the selection of cells resistant to tetracycline.

### 1. Bacterial strains and plasmids

Bacterial strains and plasmids used in this example are shown in table 5 and table 6, respectively.

**Table 5: Bacterial strains**

| **Strains** | **Genotype** | **Reference or source** |
|---|---|---|
| *E. coli* AB1157 Nal^{R} hsd⁻ | *thr1 leu6 proA2 his4, thil argE3 lacY1 galK2 ara14 xy175 mtl1 tsx33 str31 su-pE44thr⁺ hsdR⁻B nal^{R}* | M. Radman strain collection |
| *E*. *coli* MIXP1 | As AB1157 *Nal^{R} R⁻* but *mutS::Tn5 (kan^{R})* | mutS allele from M. Radman strain collection |
| *E*. *coli DH10B* | F⁻ *mcrA* Δ(*mrr⁻ hsd*RMS-mcrBC) φ80 *Iac*ZΔm15 Δ/*ac*X74 *rec*A1 *end*A1 *a*-raD139 Δ(*ara*, *leu*)7697 *ga*/U *ga*/K λ⁻ *rpsL nup*G | Invitrogene Life Technologies Cat 18290-015 |
| *B. subtilis* 1 A423 | *argGH15 leuB8 recA4 thr-5 hsd_{RI} R*⁻*M*⁻ | Bacillus Genetic stock center (Ohio State University, USA) |

**Table 6: Plasmids**

| **Plasmids** | **Selection** |
|---|---|
| *E. coli* plasmid pMIX100 | Cm^{R} |
| *B. subtilis* plasmid pMIX101 | Tc^{R}, Erm^{R} |
| transformation efficiency control plasmids pMIX 102 and pMIX 103 | Amp^{R}, TC^{R} |

### 2. Results

### 2.1 Construction of the E. coli plasmid pMIX100

The plasmid pMIX100 carries the origin of replication of pACYC184, as well as its chloramphenicol-resistance gene. pMIX100 carries also the gene *lacZ* from pBluescript SK+, which facilitates selection in cloning experiments. *lacZ* encodes for a fragment of the β-galactosidase providing α-complementation for blue/white selection of recombinants in a medium containing X-Gal and IPTG. Thus, colonies harboring pMIX100 should be blue in this medium, those carrying plasmid with a gene inserted in the polylinker (MCS) should be white. The physical map of plasmid pMIX100 is shown in figure 5.

### 2.2 Construction of the B. subtilis plasmid pMIX101

Plasmid pMIX101 is a derivative of the *B*. *subtilis* plasmid pIL253. Since the pIL253 marker *Erm^{R}*, which confers resistance to erythromycin is not useful in *E*. *coli* the tetracycline-resistance marker allowing the selection of hybrid molecules in recombination experiments was introduced. The tetracycline-resistance gene was amplified from the plasmid pACYC184. Thus, pMIX101 carries two markers: *Erm^{R}*, as a selection marker in *B. subtilis* for cloning of target genes, and *Tc^{R}*, to select recombinant hybrid molecules in *E*. *coli*. The physical map of plasmid pMIX101 is shown in figure 6.

### 2.3 Construction of the transformation efficiency control plasmids pMIX102 and pMIX103

Since the B. *subtilis* vector is not able to replicate in *E*. *coli*, a transformation efficiency control is necessary to estimate recombination frequency. Since recombinants will be selected by tetracycline resistance, the same marker must be present in the control vector. Plasmid pMIX102, a derivative of pBluescript SK+ contains the *Tc^{R}* gene amplified from pACYC184. In pMIX102 the *Tc^{R}* gene is driven by the plac promoter. The physical map of plasmid pMIX102 is shown in figure 7.

In the second control vector pMIX103 the *Tc^{R}* gene is cloned in the opposite direction than plac. Therefore this gene is expressed from its own promoter. The physical map of plasmid pMIX103 is shown in figure 8.

### 2.4 Cloning of oxa7, oxa11 and oxa5 into the E. coli plasmid pMIX100

To verify the results of the recombination experiments between 0%, 5% and 22% divergent-*oxa* genes obtained in example 1 *oxa7*, *oxa11* and *oxa5* were cloned into the *E*. *coli* plasmid pMIX100. Those genes were amplified using primers containing either *Pst*I or *Xho*I at their 5' ends. After digestion with those enzymes, the amplified DNA fragments were independently ligated with pMIX100, which was previously cut with *Pst*I + *Xho*l. Competent cells of *E*. *coli* DHB10 were electroporated with the ligation mixtures, and colonies harboring positive clones were phenotypically selected by chloramphenicol-resistance/ white color on LB plates containing Cm (30 µg/ml) + X-Gal (80 µg/ml) + IPTG (0.5 mM). For each *oxa*-cloning, five of such transformants were analyzed.

Plasmid DNAs were obtained and analysed by restriction mapping. Results confirmed that pMIX104 carries *oxa7*, pMIX106 carries *oxa11* and pMIX107 carries *oxa5*.

For cloning into the *B. subtilis* plasmid pMIX101 *oxa7* was obtained as a 0.9-kb fragment from pMIX104 by restriction with *Pst*I and *Xho*l and ligated with pMIX101, which was previously cut with the same enzymes. After transformation of *B. subtilis* 1A423 competent cells with the ligation product, cells were selected in LB containing 0.5 µg/ml of erythromycin (Erm). Plasmid DNA was obtained from 24 transformants and analysed by restriction. Results confirmed that all clones contained the 7-kb plasmid pMIX105.

The strategy for cloning the *oxa7*, *oxa11* and *oxa5* genes into the *E*. *coli* plasmid pMIX100 and into the *B. subtilis* plasmid pMIX101 is shown in figure 9.

### 2.5 In vivo recombination of oxa genes in wt compared to MutS⁻ mutant E. coli

Competent cells of *E*. *coli* AB1157 hsdR⁻ and its MMR⁻ mutant, *E*. *coli* AB1157 hsdR⁻ CΔ*mutS*, were independently transformed by electroporation with plasmids pMIX104 (*oxa7*), pMIX106 (*oxa11*) and pMIX107 (*oxa5*).

To recombine *oxa* genes, *E*. *coli* strains harboring those plasmids were electroporated with pMIX105 (*oxa7*). The *B*. *subtilis* plasmid pMIX101 was used as a negative control, whereas the *E*. *coli* vector pMIX102, carrying Tc^{R} marker, was used as the control of transformation efficiency under tetracycline selection. All DNAs were UV-irradiated prior to electroporation in order to increase recombination frequency.

Recombination frequencies were calculated by dividing the transformation efficiency obtained with *B. subtilis* plasmids per the transformation efficiency obtained with control vector pMIX92. Transformation efficiencies were in turn calculated as number of colony forming units (cfu) obtained per µg of DNA on previously described conditions.

Results obtained are summarized in table 7. In the wild-type strain, recombinants were obtained in experiments using either identical or 5% divergent *oxa* genes, whereas in the MutS⁻ mutant, recombination also happened between 22% divergent genes.

**Table 7: In vivo recombination frequencies obtained between oxa genes in both wild-type and MutS⁻ E. coli strains.**

| **Genetic divergence** **(%)** | **Recombination frequencies** | |
|---|---|---|
| | **wt strain** | **MutS⁻strain** |
| 0 | 10⁻⁵-10⁻⁶ | 10⁻⁶ |
| 5 | --- | 10⁻⁵-10⁻⁶ |
| 22 | --- | --- |

Recombination frequencies were consistent with those obtained with the former double plasmid system.

To confirm recombination, pMIX105 transformants were grown in liquid media containing tetracycline (12,5 µg/ml). Restriction analyses of plasmid DNA obtained from these cultures showed that they harbored dimers carrying *oxa*-recombinant genes, together with resident plasmids (either pMIX104 or pMIX106). Moreover, recombinant genes R1 and R2 were successfully amplified by PCR from both colonies and plasmid DNA using specific primers.

Results demonstrate that recombinants were generated with a second double plasmid system using tetracycline resistance as selective pressure.

## Claims

1. Process for generating and detecting recombinant DNA sequences in prokaryotes comprising the steps of:
a) generating a first prokaryotic cell containing an extrachromosomal recipient DNA molecule, which comprises a first DNA sequence to be recombined and which can autonomously replicate in the prokaryotic cell, and an extrachromosomal donor DNA molecule, which comprises a second DNA sequence to be recombined and at least a first marker sequence encoding a gene product and which cannot autonomously replicate in the prokaryotic cell,
b) cultivating the first prokaryotic cell under selective conditions, which force the formation of a co-integrate or hybrid molecule between the recipient and donor DNA molecules and the recombination of the two DNA sequences to be recombined and which only allow the growth and/or propagation of the cell if the gene product of the first marker sequence is expressed, and
c) isolating a second prokaryotic cell grown and/or propagated under selective conditions and containing a hybrid DNA molecule with the at least first marker sequence and a first and a second recombined DNA sequence due to recombination between the first and the second DNA sequence,
wherein the prokaryotic cell is transiently or permanently deficient in the mismatch repair system.

2. Process according to claim 1, wherein the donor DNA molecule and the recipient DNA molecules are different linear or circular DNA structures, in particular different plasmids or bacteriophages.

3. Process according to any one of claims 1 or 2, wherein the donor DNA molecule does not have an origin of replication.

4. Process according to any one of claims 1 or 2, wherein the donor DNA molecule has a non-functional origin of replication.

5. Process according to any of claim 1 to 4, wherein the donor DNA molecule is a *Bacillus subtilis* plasmid, which cannot replicate in *E*. *coli.*

6. Process according to claim 5, wherein the donor DNA molecule is the *B*. *subtilis* plasmid pMIX91 comprising the spec^{R} marker and the *phleo^{R}* marker, as deposited at the DSMZ (Ref SB202pMIX91); or the *B subtilis* plasmid pMIX101 comprising the *tc^{R}* marker as deposited at the DSMZ (Ref 1A423pMIX101).

7. Process according to any one of claims 1 to 6, wherein the first marker sequence of the donor DNA structure is selected from the group consisting of a nutritional marker, an antibiotic resistance marker and a sequence encoding a subunit of an enzyme.

8. Process according to claim 7, wherein the gene product of the first marker sequence confers resistance to an antibiotic to a cell which is sensitive to that antibiotic.

9. Process according to claim 7 or 8, wherein the first marker sequence is *spec*^{R}, the gene product of which confers to a cell resistance to spectinomycin, or *phleo^{R}*, the gene product of which confers to a cell resistance to phleomycin, or tC^{R}, the gene product of which confers to a cell resistance to tetracycline.

10. Process for generating and detecting recombinant DNA sequences in prokaryotes comprising the steps of:
d) generating a first prokaryotic cell containing an extrachromosomal recipient DNA molecule, which comprises a first DNA sequence to be recombined and which can autonomously replicate in the prokaryotic cell, and an extrachromosomal donor DNA molecule, which comprises a second DNA sequence to be recombined and at least a first marker sequence encoding a gene product and which cannot autonomously replicate in the prokaryotic cell,
e) cultivating the first prokaryotic cell under selective conditions, which force the formation of a co-integrate or hybrid molecule between the recipient and donor DNA molecules and the recombination of the two DNA sequences to be recombined and which only allow the growth and/or propagation of the cell if the gene product of the first marker sequence is expressed, and
f) isolating a second prokaryotic cell grown and/or propagated under selective conditions and containing a hybrid DNA molecule with the at least first marker sequence and a first and a second recombined DNA sequence due to recombination between the first and the second DNA sequence,
wherein the donor DNA molecule is the *B. subtilis* plasmid pMIX91 comprising the *spec^{R}* marker and the *phleo^{R}* marker, as deposited at the DSMZ (Ref SB202pMIX91); or the *B. subtilis* plasmid pMIX101 comprising the tc^{R} marker as deposited at the DSMZ (Ref 1A423pMIX101).

11. Process according to claim 10, wherein the recipient DNA molecule is a linear or circular DNA structure, in particular a plasmid or a bacteriophage.

12. Process according to claim 10 or 11, wherein the prokaryotic cell has a functional mismatch repair system.

13. Process according to claim 10 or 11, wherein the prokaryotic cell is transiently or permanently deficient in mismatch repair system.

14. Process according to any one of claims 1 to 13, wherein the recipient DNA molecule is a plasmid, which can replicate in *Escherichia coli.*

15. Process according to claim 14, wherein the recipient DNA molecule is the *E. coli* plasmid pACYC184 or the *E. coli* plasmid pMIX100 or a derivative thereof.

16. Process according to any one of claims 1 to 15, wherein the donor DNA molecule and/or its origin of replication are derived from a prokaryotic species other than the prokaryotic species in cells of which the donor DNA molecule is introduced.

17. Process according to any one of claims 1 to 16, wherein the function of the origin of replication of the donor DNA is impaired by a mutation.

18. Process according to any one of claims 1 to 17, wherein the donor DNA molecule contains a second marker sequence.

19. Process according to any one of claims 1 to 18, wherein the recipient DNA molecule contains a third marker sequence and optionally a fourth marker sequence.

20. Process according to claim 18 or 19, wherein the second, third and fourth marker sequences are protein-coding or non-coding sequences selected from the group consisting of nutritional markers, pigment markers, antibiotic resistance markers, antibiotic sensitivity markers, restriction enzymes sites, primer recognition sites and sequences encoding a subunit of an enzyme.

21. Process according to claim 20, wherein the gene products of the third and fourth marker sequence of the recipient DNA molecule confer resistance to an antibiotic to a cell which is sensitive to that antibiotic.

22. Process according to claim 21, wherein the gene product of the third marker sequence confers to a cell resistance to tetracycline.

23. Process according to claim 21, wherein the gene product of the fourth marker sequence confers to a cell resistance to chloramphenicol.

24. Process according to any one of claims 1 to 23, wherein the first and the second DNA sequences to be recombined diverge by at least two nucleotides.

25. Process according to any one of claims 1 to 24, wherein the first and the second DNA sequences to be recombined are naturally occurring sequences.

26. Process according to claim 25, wherein the first and/or the second DNA sequences to be recombined are derived from viruses, bacteria, plants, animals and/or human beings.

27. Process according to any one of claims 1 to 24, wherein the first and/or the second DNA sequences to be recombined are artificial sequences.

28. Process according to any one of claims 1 to 27, wherein each of the first and the second DNA sequences to be recombined comprises one or more protein-coding sequences and/or one or more non-coding sequences.

29. Process according to any one of claims 1 to 28, wherein the first prokaryotic cell is generated by simultaneously or sequentially introducing the recipient DNA molecule and the donor DNA molecule into a prokaryotic cell.

30. Process according to claim 29, wherein the recipient and donor DNA molecules are introduced into the prokaryotic cell via transformation, conjugation, transduction, sexduction and/or electroporation.

31. Process according to any one of claims 1 to 30, wherein the first prokaryotic cell is cultivated in the presence of at least one antibiotic to which the gene product of the first marker sequence confers resistance.

32. Process according to claim 31, wherein the first prokaryotic cell is additionally cultivated in the presence of a second, a third and/or a fourth antibiotic to which the gene products of the second marker sequence, the third marker and the fourth marker sequence, respectively, confer resistance.

33. Process according to any one of claims 1 to 32, wherein the prokaryotic cell is a cell of an archaebacterium or an eubacterium.

34. Process according to claim 33, wherein the eubacterium is a gram-negative bacterium, a gram-positive bacterium or a cyanobacterium.

35. Process according to claim 34, wherein the gram-negative bacterium is *Escherichia coli.*

36. Process according to claims 1 to 9 and 13, wherein the transient or permanent deficiency of the mismatch repair system is due to a mutation, a deletion, and/or an inducible expression or repression of one or more genes involved in the mismatch repair system, a treatment with an agent that saturates the mismatch repair system and/or a treatment with an agent that globally knocks out the mismatch repair.

37. Process according to claims 1 to 9, 13, and 36, wherein the prokaryotic cell has a mutated *mutS* gene and/or mutated *mutL* gene.

38. Process according to any one of claims 1 to 37, wherein the first and the second recombined DNA sequences contained in the hybrid DNA molecule of the second prokaryotic cell are selected and/or isolated and/or analysed.

39. Process according to claim 38, wherein the first and the second recombined DNA sequences are isolated by restriction enzyme cleavage.

40. Process according to claim 38, wherein the first and the second recombined DNA sequences are amplified by PCR.

41. Process according to any one of claims 38 to 40, wherein the isolated first and second recombined DNA sequences are inserted into a donor DNA molecule and a recipient DNA molecule, respectively, and subjected to another round of recombination.

42. *Bacillus subtilis* plasmid pMIX91 which comprises the *spec^{R}* marker and the *phleo^{R}* marker and the restriction sites *Scal, PpuMI* and EcoO109 for inserting a foreign DNA sequence, as deposited at the DSMZ (Ref SB202pMIX91).

43. *Bacillus subtilis* plasmid pMIX101 which comprises the *tc^{R}* marker sequence and the restriction sites *Xhol* and *Pstl* for inserting a foreign DNA sequence, as deposited at the DSMZ (Ref 1A423pMIX101).

44. Use of the *B. subtilis* plasmids pMIX91 or pMIX101 as claimed in claims 42 and 43 respectively, as donor DNA molecules in a process according to any one of claims 1 to 41 for generating and/or detecting recombinant DNA sequences in a prokaryotic host cell, preferably in *E*. *coli* cell.

45. Use of the *E. coli* plasmids pACYC184 or pMIX100 or a derivative thereof as recipient DNA molecule in a process according to any one of claims 1 to 41 for generating and/or detecting recombinant DNA sequences in a prokaryotic host cell, preferably in an *E*. *coli* cell.

46. Kit comprising at least a first container which comprises cells of the *E*. *coli* strain AB1157 or the *E. coli* strain MXP1 or the *E*. *coli* strain DHB10, a second container which comprises cells of the *E. coli* strain AB1157 containing plasmid pACYC184 or cells of the *E*. *coli* strain DHB10 containing plasmid pMIX100 and a third container comprising cells of the *B. subtilis* strain DSM4393 containing plasmid pMIX91, as claimed in claim 42, or cells of the *B. subtilis* strain 1A423 containing plasmid pMIX101, as claimed in claim 43.

47. Kit comprising at least a first container which comprises cells of the *E. coli* strain AB1157 or the *E. coli* strain MXP1 or the *E. coli* strain DHB10, a second container comprising DNA of plasmid pACYC184 or plasmid pMIX100 and a third container comprising DNA of plasmid pMIX91, as claimed in claim 42, or plasmid pMIX101, as claimed in claim 43.

48. A process for producing a hybrid gene and/or a protein encoded by a hybrid gene in a prokaryotic cell, wherein a process according to any one of claims 1 to 41 is carried out and the hybrid gene and/or the protein encoded by the hybrid gene is produced in the prokaryotic cell and the hybrid gene and/or the encoded protein is selected in the prokaryotic cell and/or isolated therefrom after expression.

## Patentansprüche

1. Verfahren zur Erzeugung und Erkennung rekombinanter DNA-Sequenzen in Prokaryonten, umfassend die folgenden Schritte:
a) Erzeugen einer ersten prokaryontischen Zelle, enthaltend ein extrachromosomales Empfänger-DNA-Molekül, welches eine erste DNA-Sequenz, die rekombiniert werden soll, umfasst und autonom in der prokaryontischen Zelle replizieren kann, und ein extrachromosomales Spender-DNA-Molekül, welches eine zweite DNA-Sequenz, die rekombiniert werden soll, und mindestens eine erste Markierungssequenz umfasst, die ein Genprodukt codiert, und nicht autonom in der prokaryontischen Zelle replizieren kann,
b) Züchten der ersten prokaryontischen Zelle unter selektiven Bedingungen, welche die Bildung eines Co-Integrats oder Hybridmoleküls zwischen den Empfänger- und Spender-DNA-Molekülen und die Rekombination der beiden DNA-Sequenzen, die rekombiniert werden sollen, erzwingen, und welche nur das Wachstum und/oder die Vermehrung der Zelle erlauben, wenn das Genprodukt der ersten Markersequenz exprimiert wird, und
c) Isolieren einer zweiten prokaryontischen Zelle, die unter selektiven Bedingungen gewachsen ist und/oder sich vermehrte und ein Hybrid-DNA-Molekül mit der mindestens ersten Markersequenz und einer ersten und zweiten rekombinierten DNA-Sequenz aufgrund der Rekombination zwischen der ersten und zweiten DNA-Sequenz beinhaltet,
wobei der prokaryontischen Zelle vorübergehend oder dauerhaft das Fehlpaarungs-Reparatursystem fehlt.

2. Verfahren nach Anspruch 1, wobei das Spender-DNA-Molekül und das Empfänger-DNA-Molekül unterschiedliche lineare oder ringförmige DNA-Strukturen sind, insbesondere unterschiedliche Plasmide oder Bakteriophagen.

3. Verfahren nach einem der Ansprüche 1 oder 2, wobei das Spender-DNA-Molekül keinen Replikationsursprung besitzt.

4. Verfahren nach einem der Ansprüche 1 oder 2, wobei das Spender-DNA-Molekül einen nicht funktionsfähigen Replikationsursprung besitzt.

5. Verfahren nach einem der Ansprüche 1 bis 4, wobei das Spender-DNA-Molekül ein *Bacillus subtilis*-Plasmid ist, das nicht in *E*. *coli* replizieren kann.

6. Verfahren nach Anspruch 5, wobei das Spender-DNA-Molekül das *B*. *subtilis*-Plasmid pMIX91 ist, welches die *spec*^{R}- und die *phleo^{R}-*Markierung umfasst, wie am DSMZ hinterlegt (Ref.: SB202pMIX91); oder das *B. subtilis*-Plasmid pMIX101, welches die *tc*^{R}-Markierung umfasst, wie am DSMZ hinterlegt (Ref.: 1A423pMIX101).

7. Verfahren nach einem der Ansprüche 1 bis 6, wobei die erste Markierungssequenz der Spender-DNA-Struktur ausgewählt ist aus der Gruppe bestehend aus einer Nährstoffmarkierung, einer Antibiotikaresistenz-Markierung und einer Sequenz, die eine Untereinheit eines Enzyms codiert.

8. Verfahren nach Anspruch 7, wobei das Genprodukt der ersten Markierungssequenz einer Zelle, die gegenüber einem Antibiotikum sensitiv ist, Resistenz gegenüber diesem Antibiotikum verleiht.

9. Verfahren nach Anspruch 7 oder 8, wobei die erste Markierungssequenz *spec*^{R} ist, dessen Genprodukt einer Zelle Resistenz gegenüber Spectinomycin verleiht, oder *phleo^{R},* dessen Genprodukt einer Zelle Resistenz gegenüber Phleomycin verleiht, oder *tc*^{R}, dessen Genprodukt einer Zelle Resistenz gegenüber Tetracyclin verleiht.

10. Verfahren zur Erzeugung und Erkennung rekombinanter DNA-Sequenzen in Prokaryonten, umfassend die folgenden Schritte:
d) Erzeugen einer ersten prokaryontischen Zelle, enthaltend ein extrachromosomales Empfänger-DNA-Molekül, welches eine erste DNA-Sequenz, die rekombiniert werden soll, umfasst und autonom in der prokaryontischen Zelle replizieren kann, und ein extrachromosomales Spender-DNA-Molekül, welches eine zweite DNA-Sequenz, die rekombiniert werden soll, und mindestens eine erste Markierungssequenz umfasst, die ein Genprodukt codiert, und nicht autonom in der prokaryontischen Zelle replizieren kann,
e) Züchten der ersten prokaryontischen Zelle unter selektiven Bedingungen, welche die Bildung eines Co-Integrats oder Hybridmoleküls zwischen den Empfänger- und Spender-DNA-Molekülen und die Rekombination der beiden DNA-Sequenzen, die rekombiniert werden sollen, erzwingen, und welche nur das Wachstum und/oder die Vermehrung der Zelle erlauben, wenn das Genprodukt der ersten Markersequenz exprimiert wird, und
f) Isolieren einer zweiten prokaryontischen Zelle, die unter selektiven Bedingungen gewachsen ist und/oder sich vermehrte und ein Hybrid-DNA-Molekül mit der mindestens ersten Markersequenz und einer ersten und zweiten rekombinierten DNA-Sequenz aufgrund der Rekombination zwischen der ersten und zweiten DNA-Sequenz beinhaltet,
wobei das Spender-DNA-Molekül das *B*. *subtilis*-Plasmid pMIX91 ist, welches die *spec^{R}-* und die *phleo^{R}*-Markierung umfasst, wie am DSMZ hinterlegt (Ref.: SB202pM1X91); oder das *B*. *subtilis*-Plasmid pMIX101, welches die *tc*^{R}-Markierung umfasst, wie am DSMZ hinterlegt (Ref.: 1A423pMIX101).

11. Verfahren nach Anspruch 10, wobei das Empfänger-DNA-Molekül eine lineare oder ringförmige DNA-Struktur aufweist, insbesondere ein Plasmid oder ein Bakteriophage.

12. Verfahren nach Anspruch 10 oder 11, wobei die prokaryontische Zelle ein funktionsfähiges Fehlpaarungsreparatur-System hat.

13. Verfahren nach Anspruch 10 oder 11, wobei der prokaryontischen Zelle vorübergehend oder dauerhaft das Fehlpaarungs-Reparatursystems fehlt.

14. Verfahren nach einem der Ansprüche 1 bis 13, wobei das Empfänger-DNA-Molekül ein Plasmid ist, welches in *E*. *coli* replizieren kann.

15. Verfahren nach Anspruch 14, wobei das Empfänger-DNA-Molekül das *E*. *coli*-Plasmid pACYC184 oder das *E*. *coli*-Plasmid pMIX100 oder ein Derivat davon ist.

16. Verfahren nach einem der Ansprüche 1 bis 15, wobei das Spender-DNA-Molekül und/oder sein Replikationsursprung von einer anderen prokaryontischen Spezies abstammen als die prokaryontische Spezies in Zellen, von welchen das Spender-DNA-Molekül eingebracht wurde.

17. Verfahren nach einem der Ansprüche 1 bis 16, wobei die Funktion des Replikationsursprungs der Spender-DNA durch eine Mutation beeinträchtigt ist.

18. Verfahren nach einem der Ansprüche 1 bis 17, wobei das Spender-DNA-Molekül eine zweite Markierungssequenz beinhaltet.

19. Verfahren nach einem der Ansprüche 1 bis 18, wobei das Empfänger-DNA-Molekül eine dritte Markierungssequenz und gegebenenfalls eine vierte Markierungssequenz beinhaltet.

20. Verfahren nach Anspruch 18 oder 19, wobei die zweiten, dritten und vierten Markierungssequenzen proteincodierende oder nicht-codierende Sequenzen sind ausgewählt aus der Gruppe bestehend aus Nährstoffmarkierungen, Pigmentmarkierungen, Antiobiotikaresistenz-Markierungen, Antibiotikasensitivitäts-Markierungen, Restriktionsenzymstellen, Primererkennungsstellen und Sequenzen, die eine Untereinheit eines Enzyms codieren.

21. Verfahren nach Anspruch 20, wobei die Genprodukte der dritten und vierten Markierungssequenz des Empfänger-DNA-Moleküls einer Zelle, die gegenüber einem Antibiotikum sensitiv ist, Resistenz gegenüber diesem Antibiotikum verleiht.

22. Verfahren nach Anspruch 21, wobei das Genprodukt der dritten Markierungssequenz einer Zelle Resistenz gegenüber Tetracyclin verleiht.

23. Verfahren nach Anspruch 21, wobei das Genprodukt der vierten Markierungssequenz einer Zelle Resistenz gegenüber Chloramphenicol verleiht.

24. Verfahren nach einem der Ansprüche 1 bis 23, wobei die ersten und die zweiten DNA-Sequenzen, die rekombiniert werden sollen, sich in mindestens zwei Nucleotiden unterscheiden.

25. Verfahren nach einem der Ansprüche 1 bis 24, wobei die ersten und die zweiten DNA-Sequenzen, die rekombiniert werden sollen, natürlich vorkommende Sequenzen sind.

26. Verfahren nach Anspruch 25, wobei die ersten und/oder die zweiten DNA-Sequenzen, die rekombiniert werden sollen, von Viren, Bakterien, Pflanzen, Tieren und/oder Menschen abstammen.

27. Verfahren nach einem der Ansprüche 1 bis 24, wobei die ersten und/oder die zweiten DNA-Sequenzen, die rekombiniert werden sollen, künstliche Sequenzen sind.

28. Verfahren nach einem der Ansprüche 1 bis 27, wobei jede der ersten und zweiten DNA-Sequenzen, die rekombiniert werden sollen, eine oder mehrere proteincodierende Sequenzen und/oder eine oder mehrere nicht-codierende Sequenzen umfassen.

29. Verfahren nach einem der Ansprüche 1 bis 28, wobei die erste prokaryontische Zelle erzeugt wird durch gleichzeitiges oder aufeinanderfolgendes Einbringen des Empfänger-DNA-Moleküls und des Spender-DNA-Moleküls in eine prokaryontische Zelle.

30. Verfahren nach Anspruch 29, wobei die Empfänger- und Spender-DNA-Moleküle in eine prokaryontische Zelle über Transformation, Konjugation, Transduktion, Sexduktion und/oder Elektroporation eingebracht werden.

31. Verfahren nach einem der Ansprüche 1 bis 30, wobei die erste prokaryontische Zelle in Anwesenheit von mindestens einem Antibiotikum, gegenüber welchem das Genprodukt der ersten Markierungssequenz Resistenz verleiht, gezüchtet wird.

32. Verfahren nach Anspruch 31, wobei die erste prokaryontische Zelle zusätzlich in Anwesenheit eines zweiten, eines dritten und/oder eines vierten Antibiotikums, gegenüber welchem die Genprodukte der zweiten, dritten beziehungsweise vierten Markierungssequenz Resistenz verleihen, gezüchtet wird.

33. Verfahren nach einem der Ansprüche 1 bis 32, wobei die prokaryontische Zelle eine Zelle eines Archaebakteriums oder eines Eubakteriums ist.

34. Verfahren nach Anspruch 33, wobei das Eubakterium ein gramnegatives Bakterium, ein grampositives Bakterium oder ein Cyanobakterium ist.

35. Verfahren nach Anspruch 34, wobei das gramnegative Bakterium *Escherichia coli* ist.

36. Verfahren nach den Ansprüchen 1 bis 9 und 13, wobei die vorübergehende oder dauerhafte Mangelfunktion des Fehlpaarungsreparatur-Systems durch eine Mutation, eine Deletion und/oder eine induzierbare Expression oder Repression von einem oder mehreren Genen, die am Fehlpaarungsreparatur-System beteiligt sind, eine Behandlung mit einem Stoff, der das Fehlpaarungsreparatur-System sättigt, und/oder eine Behandlung mit einem Stoff, der global die Fehlpaarungsreparatur ausschaltet, zustande kommt.

37. Verfahren nach den Ansprüchen 1 bis 9, 13 und 36, wobei die prokaryontische Zelle ein mutiertes *mutS*-Gen und/oder ein mutiertes *mutL*-Gen hat.

38. Verfahren nach einem der Ansprüche 1 bis 37, wobei die ersten und zweiten rekombinierten DNA-Sequenzen, die im Hybrid-DNA-Molekül der zweiten prokayrotischen Zelle beinhaltet sind, ausgewählt und/oder isoliert und/oder analysiert werden.

39. Verfahren nach Anspruch 38, wobei die ersten und zweiten rekombinierten DNA-Sequenzen durch Schneiden mit Restriktionsenzymen isoliert werden.

40. Verfahren nach Anspruch 38, wobei die ersten und zweiten rekombinierten DNA-Sequenzen durch PCR amplifiziert werden.

41. Verfahren nach einem der Ansprüche 38 bis 40, wobei die isolierten ersten und zweiten rekombinierten DNA-Sequenzen in ein Spender-DNA-Molekül beziehungsweise ein Empfänger-DNA-Molekül eingefügt werden und einer weitern Rekombinationsrunde unterworfen werden.

42. *Bacillus* subtilis-Plasmid pMIX91, welches die *spec*^{R}-Markierung und die *phleo*^{R}-Markierung und die Restriktionsstellen *Scal, PpuMI* und *EcoO*109 umfasst, für die Einfügung einer fremden DNA-Sequenz, wie am DSMZ hinterlegt (Ref.: SB202pMIX91).

43. *Bacillus* subtilis-Plasmid pMIX101, welches die *tc*^{R}-Markierungssequenz und die Restriktionsstellen Xhol und *Pstl* umfasst, für die Einfügung einer fremden DNA-Sequenz, wie am DSMZ hinterlegt (Ref.: 1A423pMIX101).

44. Verwendung des *B*. *subtilis*-Plasmids pM1X91 oder pM1X101 wie beansprucht in Ansprüchen 42 beziehungsweise 43, als Spender-DNA-Moleküle in einem Verfahren nach einem der Ansprüche 1 bis 41 zur Erzeugung und/oder Erkennung rekombinanter DNA-Sequenzen in einer prokaryontischen Wirtszelle, vorzugsweise in einer *E*. *coli*-Zelle.

45. Verwendung des *E*. *coli*-Plasmids pACYC184 oder pMIX100 oder eines Derivats davon als Empfänger-DNA-Molekül in einem Verfahren nach einem der Ansprüche 1 bis 41 zur Erzeugung und/oder Erkennung rekombinanter DNA-Sequenzen in einer prokaryontischen Wirtszelle, vorzugsweise in einer *E*. *coli*-Zelle.

46. Kit, umfassend mindestens einen ersten Behälter, umfassend Zellen des *E*. *coli*-Stamms AB1157 oder des *E*. *coli*-Stamms MXP1 oder des *E*. *coli*-Stamms DHB10, einen zweiten Behälter, umfassend Zellen des *E*. *coli*-Stamms AB1157 beinhaltend das Plasmid pACYC184 oder Zellen des *E*. *coli*-Stamms DHB10 beinhaltend das Plasmid pMIX100, und einen dritten Behälter, umfassend Zellen des *B*. *subtilis*-Stamms DSM4393 beinhaltend das Plasmid pMIX91, wie beansprucht in Anspruch 42, oder Zellen des *B*. *subtilis*-Stamms 1A423 beinhaltend das Plasmid pMIX101, wie beansprucht in Anspruch 43.

47. Kit, umfassend mindestens einen ersten Behälter, umfassend Zellen des *E*. *coli*-Stamms AB1157 oder des *E*. *coli*-Stamms MXP1 oder des *E*. *coli*-Stamms DHB10, einen zweiten Behälter, umfassend DNA des Plasmids pACYC184 oder des Plasmids pMIX100, und einen dritten Behälter, umfassend DNA des Plasmids pMIX91, wie beansprucht in Anspruch 42, oder des Plasmids pMIX101, wie beansprucht in Anspruch 43.

48. Verfahren zur Herstellung eines Hybridgens und/oder eines Proteins, das von einem Hybridgen codiert wird, in einer prokaryontischen Zelle, wobei ein Verfahren nach einem der Ansprüche 1 bis 41 ausgeführt wird und das Hybridgen und/oder das Protein, das von dem Hybridgen codiert wird, in der prokaryontischen Zelle hergestellt werden und das Hybridgen und/oder das Protein, das von dem Hybridgen codiert wird, in der prokaryotischen Zelle ausgewählt werden und/oder daraus nach der Expression isoliert werden.

## Revendications

1. Procédé pour engendrer et détecter des séquences d'ADN recombinant dans des procaryotes, comprenant les étapes consistant :
a) à engendrer une première cellule procaryotique contenant une molécule d'ADN extrachromosomique servant de receveur, qui comprend une première séquence d'ADN à recombiner et qui peut se répliquer de manière autonome dans la cellule procaryotique, une molécule d'ADN extrachromosomique servant de donneur, qui comprend une seconde séquence d'ADN à recombiner et au moins une première séquence de marqueur codant pour un produit de gène et qui ne peut se répliquer de manière autonome dans la cellule procaryotique,
b) à cultiver la première cellule procaryotique dans des conditions sélectives, qui contraignent à la formation d'un produit de co-intégration ou d'une molécule hybride entre la molécule d'ADN servant de receveur et la molécule d'ADN servant de donneur et la recombinaison des deux séquences d'ADN à recombiner et qui permettent seulement la croissance et/ou la multiplication de la cellule si le produit de gène de la première séquence de marqueur est exprimé, et
c) à isoler une seconde cellule procaryotique cultivée et/ou multipliée dans des conditions sélectives et contenant une molécule d'ADN hybride avec ladite au moins une première quantité de marqueur et des première et seconde séquences d'ADN recombinées en raison de la recombinaison entre les première et seconde séquences d'ADN,
dans lequel la cellule procaryotique est déficiente de manière transitoire ou permanente en le système de réparation de mésappariement.

2. Procédé suivant la revendication 1, dans lequel la molécule d'ADN servant de donneur et les molécules d'ADN servant de receveur sont des structures d'ADN linéaires ou circulaires, en particulier des plasmides ou bactériophages différents.

3. Procédé suivant l'une quelconque des revendications 1 et 2, dans lequel la molécule d'ADN servant de donneur ne possède pas d'origine de réplication.

4. Procédé suivant l'une quelconque des revendications 1 et 2, dans lequel la molécule d'ADN servant de donneur a une origine de réplication non fonctionnelle.

5. Procédé suivant l'une quelconque des revendications 1 à 4, dans lequel la molécule d'ADN servant de donneur est un plasmide de *Bacillus subtilis,* qui ne peut se répliquer dans *E*. *coli.*

6. Procédé suivant la revendication 5, dans lequel la molécule d'ADN servant de donneur est le plasmide pMIX91 de *B. subtilis* comprenant le marqueur *spec^{R}* et le marqueur *phleo^{R},* tel que déposé au DSMZ (référence SB202pMIX91) ; ou le plasmide pMIX101 de *B. subtilis* comprenant le marqueur *tc^{R}* tel que déposé au DSMZ (référence 1A423pMIX101).

7. Procédé suivant l'une quelconque des revendications 1 à 6, dans lequel la première séquence de marqueur de la structure d'ADN servant de donneur est choisie dans le groupe consistant en un marqueur nutritionnel, un marqueur de résistance, un antibiotique et une séquence codant pour une sous-unité d'une enzyme.

8. Procédé suivant la revendication 7, dans lequel le produit de gène de la première séquence de marqueur confère une résistance à un antibiotique à une cellule qui est sensible à cet antibiotique.

9. Procédé suivant la revendication 7 ou 8, dans lequel la première séquence de marqueur est la séquence *spee^{R},* dont le produit de gène confère à une cellule une résistance à la spectinomycine, ou la séquence *phleo^{R},* dont le produit de gène confère à une cellule une résistance à la phléomycine, ou bien la séquence tc^{R}, dont le produit de gène confère à une cellule une résistance à la tétracycline.

10. Procédé pour engendrer et détecter des séquences d'ADN recombinant dans des procaryotes, comprenant les étapes consistant :
d) à engendrer une première cellule procaryotique contenant une molécule d'ADN extrachromosomique servant de receveur, qui comprend une première séquence d'ADN à recombiner et qui peut se répliquer de manière autonome dans la cellule procaryotique, une molécule d'ADN extrachromosomique servant de donneur, qui comprend une seconde séquence d'ADN à recombiner et au moins une première séquence de marqueur codant pour un produit de gène et qui ne peut se répliquer de manière autonome dans la cellule procaryotique,
e) à cultiver la première cellule procaryotique dans des conditions sélectives, qui contraignent à la formation d'un produit de co-intégration ou d'une molécule hybride entre la molécule d'ADN servant de receveur et de donneur et la recombinaison des deux séquences d'ADN à recombiner et qui permettent seulement la croissance et/ou la multiplication de la cellule si le produit de gène de la première séquence de marqueur est exprimé, et
f) à isoler une seconde cellule procaryotique cultivée et/ou multipliée dans des conditions sélectives et contenant une molécule d'ADN hybride avec ladite au moins une première séquence de marqueur et des première et seconde séquences d'ADN recombinées en raison de la recombinaison entre les première et seconde séquences d'ADN,
dans lequel la molécule d'ADN servant de donneur est le plasmide pMIX91 de *B. subtilis* comprenant le marqueur *spec^{R}* et le marqueur *phleo^{R},* tel que déposé au DSMZ (référence SB202pMIX91) ; ou le plasmide pMIX101 de *B. subtilis* comprenant le marqueur tc^{R} tel que déposé au DSMZ (référence 1A423pMIX101).

11. Procédé suivant la revendication 10, dans lequel la molécule d'ADN servant de receveur est une structure d'ADN linéaire ou circulaire, en particulier un plasmide ou un bactériophage.

12. Procédé suivant la revendication 10 ou 11, dans lequel la cellule procaryotique possède un système fonctionnel de réparation de mésappariement.

13. Procédé suivant la revendication 10 ou 11, dans lequel la cellule procaryotique est déficiente de manière transitoire ou permanente en système de réparation de mésappariement.

14. Procédé suivant l'une quelconque des revendications 1 à 13, dans lequel la molécule d'ADN servant de receveur est un plasmide, qui peut se répliquer dans *Escherichia coli.*

15. Procédé suivant la revendication 14, dans lequel la molécule d'ADN servant de receveur est le plasmide pACYC184 de *E*. *coli* ou le plasmide pMIX100 de *E*. *coli* ou un de leurs dérivés.

16. Procédé suivant l'une quelconque des revendications 1 à 15, dans lequel la molécule d'ADN servant de donneur et/ou son origine de réplication sont dérivées d'une espèce procaryotique autre que l'espèce procaryotique dans les cellules de laquelle la molécule d'ADN servant de donneur est introduite.

17. Procédé suivant l'une quelconque des revendications 1 à 16, dans lequel la fonction de l'origine de réplication de l'ADN servant de donneur est entravée par une mutation.

18. Procédé suivant l'une quelconque des revendications 1 à 17, dans lequel la molécule d'ADN servant de donneur contient une seconde séquence de marqueur.

19. Procédé suivant l'une quelconque des revendications 1 à 18, dans lequel la molécule d'ADN servant de receveur contient une troisième séquence de marqueur et éventuellement une quatrième séquence de marqueur.

20. Procédé suivant la revendication 18 ou 19, dans lequel les deuxième, troisième et quatrième séquences de marqueurs sont des séquences codant ou ne codant pas pour des protéines choisies dans le groupe consistant en des marqueurs nutritionnels, des marqueurs de pigment, des marqueurs de résistance à des antibiotiques, des marqueurs de sensibilité à des antibiotiques, des sites d'enzymes de restriction, des sites de reconnaissance d'amorces et des séquences codant pour une sous-unité d'une enzyme.

21. Procédé suivant la revendication 20, dans lequel les produits de gène des troisième et quatrième séquences de marqueurs de la molécule d'ADN servant de receveur confèrent une résistance à un antibiotique à une cellule qui est sensible à cet antibiotique.

22. Procédé suivant la revendication 21, dans lequel le produit de gène de la troisième séquence de marqueur confère à une cellule une résistance à la tétracycline.

23. Procédé suivant la revendication 21, dans lequel le produit de gène de la quatrième séquence de marqueur confère à une cellule une résistance au chloramphénicol.

24. Procédé suivant l'une quelconque des revendications 1 à 23, dans lequel les première et seconde séquences d'ADN à recombiner divergent par au moins deux nucléotides.

25. Procédé suivant l'une quelconque des revendications 1 à 24, dans lequel les première et seconde séquences à recombiner sont des séquences naturelles.

26. Procédé suivant la revendication 25, dans lequel les première et/ou seconde séquences d'ADN à recombiner sont dérivées de virus, de bactéries, de plantes, d'animaux et/ou d'êtres humains.

27. Procédé suivant l'une quelconque des revendications 1 à 24, dans lequel les première et/ou seconde séquences d'ADN à recombiner sont des séquences artificielles.

28. Procédé suivant l'une quelconque des revendications 1 à 27, dans lequel chacune des première et seconde séquences d'ADN à recombiner comprend une ou plusieurs séquences codant pour des protéines et/ou une ou plusieurs séquences non codantes.

29. Procédé suivant l'une quelconque des revendications 1 à 28, dans lequel la première cellule procaryotique est engendrée en introduisant simultanément ou séquentiellement la molécule d'ADN servant de receveur et la molécule d'ADN servant de donneur dans une cellule procaryotique.

30. Procédé suivant la revendication 29, dans lequel les molécules d'ADN servant de receveur et de donneur sont introduites dans la cellule procaryotique par transformation, conjugaison, transduction, sexduction et/ou électroporation.

31. Procédé suivant l'une quelconque des revendications 1 à 30, dans lequel la première cellule procaryotique est cultivée en présence d'au moins un antibiotique auquel le produit de gène de la première séquence de marqueur confère une résistance.

32. Procédé suivant la revendication 31, dans lequel la première cellule procaryotique est cultivée en outre en présence d'un second, troisième et/ou quatrième antibiotiques auxquels les produits de gène de la deuxième séquence de marqueur, du troisième marqueur et de la quatrième séquence de marqueur, respectivement, confèrent une résistance.

33. Procédé suivant l'une quelconque des revendications 1 à 32, dans lequel la cellule procaryotique est une cellule d'une archaebactérie ou d'une eubactérie.

34. Procédé suivant la revendication 33, dans lequel l'eubactérie est une bactérie Gram-négative, une bactérie Gram-positive ou une cyanobactérie.

35. Procédé suivant la revendication 34, dans lequel la bactérie Gram-négative est *Escherichia coli.*

36. Procédé suivant les revendications 1 à 9 et 13, dans lequel la déficience transitoire ou permanente du système de réparation de mésappariement est due à une mutation de délétion et/ou une expression ou répression inductible d'un ou plusieurs gènes impliqués dans le système de réparation de mésappariement, un traitement avec un agent qui sature le système de réparation de mésappariement et/ou un traitement avec un agent qui inactive globalement la réparation de mésappariement.

37. Procédé suivant les revendications 1 à 9, 13 et 36, dans lequel la cellule procaryotique possède un gène *mutS* muté et/ou un gène *mutL* muté.

38. Procédé suivant l'une quelconque des revendications 1 à 37, dans lequel les première et seconde séquences d'ADN recombinées présentes dans la molécule d'ADN hybride de la seconde cellule procaryotique sont sélectionnées et/ou isolées et/ou analysées.

39. Procédé suivant la revendication 38, dans lequel les première et seconde séquences d'ADN recombinées sont isolées par clivage avec des enzymes de restriction.

40. Procédé suivant la revendication 38, dans lequel les première et seconde séquences d'ADN recombinées sont amplifiées par PCR.

41. Procédé suivant l'une quelconque des revendications 38 à 40, dans lequel les première et seconde séquences d'ADN recombinées isolées sont insérées dans une molécule d'ADN servant de donneur et une molécule d'ADN servant de receveur, respectivement, et sont soumises à un autre cycle de recombinaison.

42. Plasmide pMIX91 de *Bacillus subtilis* qui comprend le marqueur *spec^{R}* et le marqueur *phleo^{R}* et les sites de restriction *ScaI, PpuMI* et *Eeo*O109 pour l'insertion d'une séquence d'ADN étrangère, tel que déposé au DSMZ (référence SB202pMIX91).

43. Plasmide pMIX101 de *Bacillus subtilis* qui comprend la séquence de marqueur *tc^{R}* et les sites de restriction *XhoI* et *PstI* pour l'insertion d'une séquence d'ADN étrangère, tel que déposé au DSMZ (référence 1A423pMIX101).

44. Utilisation des plasmides pMIX91 ou pMIX101 de *B. subtilis* suivant les revendications 42 et 43, respectivement, comme molécules d'ADN servant de donneur dans un procédé suivant l'une quelconque des revendications 1 à 41 pour engendrer et/ou détecter des séquences d'ADN recombinant dans une cellule hôte procaryotique, de préférence dans une cellule de *E. coli.*

45. Utilisation des plasmides pACYC184 ou pMIX100 de *E. coli* ou d'un de leurs dérivés comme molécules d'ADN servant de receveur dans un procédé suivant l'une quelconque des revendications 1 à 41 pour engendrer et/ou détecter des séquences d'ADN recombinant dans une cellule hôte procaryotique, de préférence dans une cellule de *E*. *coli.*

46. Kit comprenant au moins un premier récipient qui comprend des cellules de la souche de *E*. *coli* AB1157 ou de la souche de *E*. *coli* MXP1 ou bien de la souche de *E*. *coli* DHB10, un deuxième récipient qui comprend des cellules de la souche de *E*. *coli* AB1157 contenant le plasmide pACYC184 ou des cellules de la souche de *E*. *coli* DHB10 contenant le plasmide pMIX100 et un troisième récipient comprenant des cellules de la souche de *B. subtilis* DSM4393 contenant le plasmide pMIX91, suivant la revendication 42, ou des cellules de la souche de *B. subtilis* 1A423 contenant le plasmide pMIX101, suivant la revendication 43.

47. Kit comprenant au moins un premier récipient qui comprend des cellules de la souche de *E*. *coli* AB1157 ou de la souche de *E. coli* MXP1 ou bien de la souche de *E. coli* DHB10, un deuxième récipient comprenant l'ADN du plasmide PACYC184 ou du plasmide pMIX100 et un troisième récipient comprenant l'ADN du plasmide pMIX91, suivant la revendication 42, ou du plasmide pMIX101, suivant la revendication 43.

48. Procédé pour la production d'un gène hybride et/ou d'une protéine codée par un gène hybride dans une cellule procaryotique, dans lequel un procédé suivant l'une quelconque des revendications 1 à 41 est mis en oeuvre et le gène hybride et/ou la protéine codée par le gène hybride est produit dans la cellule procaryotique et le gène hybride et/ou la protéine codée est sélectionné dans la cellule procaryotique et/ou isolé de cette cellule après expression.
